# EUROPEAN PATENT APPLICATION

(11) **EP 4 520 769 A1**
(43) Date of publication of application: **12.03.2025**
(21) Application number: 23778441.8
(22) Date of filing: 31.03.2023
(51) Int. Cl.: C07K 16/28, C12N 15/13, A61K 39/395, A61P 35/00

(54) **ANTI-PD-1 MONOCLONAL ANTIBODY, DERIVATIVE THEREOF AND USE THEREOF**

(30) Priority: 02.04.2022 CN 202210345658
(71) Applicant: Biotheus Inc., Tangjiawan Town, Xiangzhou District Zhuhai, Guangdong 519080 (CN)
(72) Inventor: ZHAI, Tianhang, Zhuhai, Guangdong 519080 (CN); HUANG, Weifeng, Zhuhai, Guangdong 519080 (CN); PENG, Shaogang, Zhuhai, Guangdong 519080 (CN); CHEN, Yuanhong, Zhuhai, Guangdong 519080 (CN); YAN, Yao, Zhuhai, Guangdong 519080 (CN); TSUN, Andy, Zhuhai, Guangdong 519080 (CN); PAULI, Noel, Zhuhai, Guangdong 519080 (CN); NIERSEN, Nels, Zhuhai, Guangdong 519080 (CN)
(74) Representative: Maiwald GmbH
(86) International application number: PCT/CN2023/085413
(87) International publication number: WO 2023/186077

(57) **Abstract**

The present invention relates to the technical field of biomedicine or biopharmaceutics, and more specifically to an anti-PD-1 monoclonal antibody, a derivative thereof and use thereof.

## Description

### Technical Field

The present invention relates to the field of biomedicine or biopharmaceutical technology, and more specifically to an anti-PD-1 monoclonal antibody and its derivative and use.

### Background Art

Programmed cell death protein-1 (PD-1, also known as CD279) is usually expressed on activated lymphocytes and further upregulated on tumor-infiltrating lymphocytes. PD-1 can inhibit the overactivation and proliferation of T cells by binding to the ligands PD-L1 and PD-L2 highly expressed on tumor cells and antigen-presenting cells, exert negative immunoregulatory effects, and mediate tumor immune escape.

At present, a number of anti-PD-1 monoclonal antibodies have been approved for the treatment of various tumors (e.g., classical Hodgkin's lymphoma, squamous or non-squamous NSCLC, hepatocellular carcinoma, nasopharyngeal carcinoma, urothelial carcinoma, esophageal squamous cell carcinoma, melanoma, etc.). However, the approved indications of anti-PD-1 monoclonal antibodies are mostly focused on certain tumors that are sensitive to immunotherapy, and the objective remission rate of monotherapy for most tumors is low (mostly less than 30%). One of the important reasons may be that there are few immune cells infiltrating in tumor tissues and/or a high proportion of immunosuppressive cells (e.g., Treg, MDSC), as well as the participation of other immunosuppressive pathways.

Therefore, it is urgent and necessary to develop anti-PD-1 antibodies or their derivatives with better clinical efficacy, which will provide more medication options for patients with diseases such as cancers and infections.

### Contents of the Invention

After intensive research and creative working, the inventors have obtained a new anti-PD-1 antibodies and their derivatives. The inventors surprisingly found that the anti-PD-1 antibodies and their derivatives of the present invention have good affinity and biological activity, and have anti-tumor potential. The following invention is thus provided:

### Antibody of the present invention

Therefore, in one aspect, the present invention provides an antibody or an antigen-binding fragment thereof capable of specifically binding to PD-1, wherein the antibody or antigen-binding fragment thereof comprises:
a heavy chain variable region (VH) comprising the following 3 complementary determining regions (CDRs): VH CDR1 having a sequence as set forth in SEQ ID NO: 7 or 13, VH CDR2 having a sequence as set forth in SEQ ID NO: 8, and VH CDR3 having a sequence as set forth in SEQ ID NO: 9; and/or,
a light chain variable region (VL) comprising the following 3 complementary determining regions (CDRs): VL CDR1 having a sequence as set forth in SEQ ID NO: 10, VL CDR2 having a sequence as set forth in SEQ ID NO: 11 or 14, and VL CDR3 having a sequence as set forth in SEQ ID NO: 12.

In certain embodiments, the antibody or antigen-binding fragment thereof comprises: 3 CDRs as contained in the VH as set forth in SEQ ID NO: 1, 3 or 5, and/or, 3 CDRs as contained in the VL as set forth in SEQ ID NO: 2, 4 or 6. In certain embodiments, the CDRs are identified by the Kabat numbering system, the Chothia numbering system, or the IMGT numbering system.

In certain embodiments, the antibody or antigen-binding fragment thereof comprises:
(1) 3 CDRs as contained in the VH as set forth in SEQ ID NO: 1, and/or 3 CDRs as contained in the VL as set forth in SEQ ID NO: 2;
(2) 3 CDRs as contained in the VH as set forth in SEQ ID NO: 3, and/or 3 CDRs as contained in the VL as set forth in SEQ ID NO: 4; or
(3) 3 CDRs as contained in the VH as set forth in SEQ ID NO: 5, and/or 3 CDRs as contained in the VL as set forth in SEQ ID NO: 6.

In certain embodiments, the antibody or antigen-binding fragment thereof comprises: a heavy chain variable region (VH) comprising the following 3 complementary determining regions (CDRs): VH CDR1 having a sequence as set forth in SEQ ID NO: 7, VH CDR2 having a sequence as set forth in SEQ ID NO: 8, and VH CDR3 having a sequence as set forth in SEQ ID NO: 9; and/or, a light chain variable region (VL) comprising the following 3 complementary determining regions (CDRs): VL CDR1 having a sequence as set forth in SEQ ID NO: 10, VL CDR2 having a sequence as set forth in SEQ ID NO: 11, and VL CDR3 having a sequence as set forth in SEQ ID NO: 12.

In certain embodiments, the antibody or antigen-binding fragment thereof comprises: a heavy chain variable region (VH) comprising the following 3 complementary determining regions (CDRs): VH CDR1 having a sequence as set forth in SEQ ID NO: 13, VH CDR2 having a sequence as set forth in SEQ ID NO: 8, and VH CDR3 having a sequence as set forth in SEQ ID NO: 9; and/or, a light chain variable region (VL) comprising the following 3 complementary determining regions (CDRs): VL CDR1 having a sequence as set forth in SEQ ID NO: 10, VL CDR2 having a sequence as set forth in SEQ ID NO: 14, and VL CDR3 having a sequence as set forth in SEQ ID NO: 12.

In certain embodiments, the antibody or antigen-binding fragment thereof comprises: a heavy chain variable region (VH) comprising a sequence as set forth in SEQ ID NO: 1, 3, or 5, or variant thereof; and/or, a light chain variable region (VL) comprising a sequence as set forth in SEQ ID NO: 2, 4, or 6, or variant thereof.

In certain embodiments, the antibody or antigen-binding fragment thereof comprises: a heavy chain variable region (VH) comprising a sequence as set forth in SEQ ID NO: 1 or 3, or variant thereof; and/or, a light chain variable region (VL) comprising a sequence as set forth in SEQ ID NO: 2 or 4, or variant thereof.

In certain embodiments, the antibody or antigen-binding fragment thereof comprises: a heavy chain variable region (VH) comprising a sequence as set forth in SEQ ID NO: 1 or variant thereof; and/or, a light chain variable region (VL) comprising a sequence as set forth in SEQ ID NO: 2 or variant thereof.

In certain embodiments, the antibody or antigen-binding fragment thereof comprises: a heavy chain variable region (VH) comprising a sequence as set forth in SEQ ID NO: 3 or variant thereof; and/or, a light chain variable region (VL) comprising a sequence as set forth in SEQ ID NO: 4 or variant thereof.

In certain embodiments, the antibody or antigen-binding fragment thereof comprises: a heavy chain variable region (VH) comprising a sequence as set forth in SEQ ID NO: 5 or variant thereof; and/or, a light chain variable region (VL) comprising a sequence as set forth in SEQ ID NO: 6 or variant thereof.

The variant as described in any of the above embodiments has a substitution, deletion or addition of one or more amino acids (e.g., a substitution, deletion or addition of 1, 2, 3, 4 or 5 amino acids), or a sequence having a sequence identity of at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived; preferably, the substitution is a conservative substitution.

In certain embodiments, the antibody or antigen-binding fragment thereof comprises:
(1) a VH comprising a sequence as set forth in SEQ ID NO: 1 and a VL comprising a sequence as set forth in SEQ ID NO: 2;
(2) a VH comprising a sequence as set forth in SEQ ID NO: 3 and a VL comprising a sequence as set forth in SEQ ID NO: 4; or,
(3) a VH comprising a sequence as set forth in SEQ ID NO: 5 and a VL comprising a sequence as set forth in SEQ ID NO: 6.

In certain embodiments, the antibody or antigen-binding fragment thereof in any of the above embodiments may further comprise a constant region sequence derived from a mammalian (e.g., mouse or human) immunoglobulin.

In certain embodiments, the heavy chain of the antibody or antigen-binding fragment thereof in any of the above embodiments comprises a heavy chain constant region derived from a mouse or human immunoglobulin (e.g., IgG1, IgG2, IgG3, or IgG4). In certain embodiments, the light chain of the antibody or antigen-binding fragment thereof in any of the above embodiments comprises a light chain constant region derived from a mouse or human immunoglobulin (e.g., κ or λ).

In certain embodiments, the heavy chain constant region is an IgG heavy chain constant region, such as an IgG1, IgG2, IgG3, or IgG4 heavy chain constant region.

In certain embodiments, the heavy chain constant region (CH) has the same or substantially the same effector function as a wild-type heavy chain constant region sequence.

In certain embodiments, the heavy chain constant region (CH) may comprise one or more amino acid mutations or chemical modifications to alter one or more of the following properties of the antibody of the present invention: Fc receptor binding, antibody glycosylation, number of cysteine residues, effector cell function, or complement function, etc. Functional changes can be produced by replacing at least one amino acid residue in the constant region of the antibody with a different residue or chemical modification, for example, changing the affinity of the antibody for an effector ligand (e.g., FcR or complement C1q), thereby changing (e.g., reducing or enhancing) the effector function. The Fc region of the antibody mediates several important effector functions, such as ADCC, phagocytosis, CDC, etc.

In certain embodiments, the heavy chain constant region (CH) has reduced or eliminated ADCC activity, such as comprising a LALA mutation (L234A, L235A).

In certain embodiments, the antibody or antigen-binding fragment thereof comprises a heavy chain constant region (CH) as set forth in SEQ ID NO: 15 and/or a light chain constant region (CL) as set forth in SEQ ID NO: 16.

In certain embodiments, the antigen-binding fragment in any of the above embodiments can be selected from Fab, Fab', (Fab')₂, Fv, disulfide-bonded Fv, scFv, and diabody.

In certain embodiments, the antibody in any of the above embodiments is a chimeric antibody, a humanized antibody, a bispecific antibody, or a multispecific antibody.

### scFv

In certain embodiments, the antigen-binding fragment in any of the above embodiments is an scFv. It is known in the art that the stability of the scFv structure can be enhanced by forming interchain disulfide bonds. Therefore, in certain embodiments, the scFv comprises a disulfide bond. Methods for introducing disulfide bonds in scFv are well known to those skilled in the art, for example, by introducing cysteine (C) in the VH and VL of the scFv, respectively. In certain embodiments, the VH and VL of the first antigen-binding domain each comprise a residue in the FR region that is mutated to cysteine (C), so that the scFv formed by the VH and VL may comprise a disulfide bond. In certain embodiments, a residue in the FR2 region of the VH of the first antigen-binding domain and a residue in the FR4 region of the VL are mutated to cysteine (C).

In certain embodiments, the scFv preferably comprises the VH as set forth in SEQ ID NO: 3 and the VL as set forth in SEQ ID NO: 4, or comprises the VH as set forth in SEQ ID NO: 5 and the VL as set forth in SEQ ID NO: 6. In such embodiments, the scFv comprises an interchain disulfide bond.

In certain embodiments, the scFv has a structure shown as VH-L-VL or VL-L-VH, wherein L is a peptide linker. In certain embodiments, L is a peptide linker comprising one or more glycine (G) and/or one or more serine (S) and/or one or more alanine (A), such as a flexible peptide comprising (G₄S)ₙ or (G₄A)ₙ, n=1, 2, 3 or 4. In certain exemplary embodiments, L is (G₄S)₄.

In certain embodiments, the scFv may further comprise an additional biologically active polypeptide in its N segment and/or C segment to extend its half-life to form a polypeptide construct. In certain embodiments, the additional biologically active polypeptide is an immunoglobulin Fc domain.

In certain embodiments, the immunoglobulin Fc domain is optionally connected to the N-terminal or C-terminal (e.g., N-terminal) of the scFv via a peptide linker. In certain embodiments, the peptide linker comprises one or more glycine (G) and/or one or more serine (S) and/or one or more alanine (A), such as a flexible peptide comprising (G₄S)ₙ or (G₄A)ₙ, wherein n=1, 2, 3 or 4. In certain exemplary embodiments, the peptide linker is (G₄S)₂ or (G₄A)₂.

In certain embodiments, the immunoglobulin Fc domain is an Fc domain of IgG (e.g., an Fc domain of IgG1).

In certain embodiments, the immunoglobulin Fc domain comprises the sequence as set forth in SEQ ID NO: 22.

In certain exemplary embodiments, the scFv comprises the sequence as set forth in SEQ ID NO: 23 or 24.

In certain embodiments, the antibody or antigen-binding fragment thereof of the present invention possess at least one of the following biological functions:
(1) specifically recognizing/binding to PD-1 (especially human PD-1);
(2) blocking the binding of PD-1 to PD-L1, or inhibiting and/or blocking intracellular signal transduction mediated by PD-1 binding to PD-L1;
(3) improving the activity of immune cells (e.g., T cells) in vitro and in vivo, for example, increasing the secretion level of effector cytokines (e.g., IL-2, IFN-γ, etc.), proliferation activity, expression level of activation markers (e.g., CD25, CD69, etc.), and/or cell killing activity;
(4) enhancing immune response (e.g., T cell-mediated immune response) in vitro and in vivo;
(5) preventing and/or treating a tumor or infection in a subject.

Herein, the antibody or antigen-binding fragment thereof of the present invention may comprise a variant that differs from the antibody or antigen-binding fragment thereof from which it is derived only in a conservative substitution of one or more amino acid residues (e.g., a conservative substitution of up to 20, up to 15, up to 10, or up to 5 amino acids), or have a sequence identity of at least 85%, at least 90%, at least 95%, at least 96%, at least 97%, at least 98%, at least 99%, or 100% as compared with the antibody or antigen-binding fragment thereof from which it is derived, and substantially retain the above-mentioned biological functions of the antibody or antigen-binding fragment thereof from which it is derived.

### Derivatized antibody

The antibody or antigen-binding fragment thereof of the present invention may be derivatized, for example, linked to another molecule (e.g., another polypeptide or protein). Generally, derivatization (e.g., labeling) of antibody or antigen-binding fragment thereof does not adversely affect its binding to PD-1 (particularly human PD-1). Therefore, the antibody or antigen-binding fragment thereof of the present invention is also intended to include such derivatized forms. For example, the antibody or antigen-binding fragment thereof of the present invention may be functionally linked (by chemical coupling, genetic fusion, non-covalent bonding, or other methods) to one or more other molecular groups, such as another antibody (e.g., to form a bispecific antibody), a detection agent, a pharmaceutical agent, and/or a protein or polypeptide that can mediate the binding of the antibody or antigen-binding fragment to another molecule (e.g., an avidin or polyhistidine tag). In addition, the antibody or antigen-binding fragment thereof of the present invention may be derivatized with a chemical group, such as polyethylene glycol (PEG), methyl or ethyl, or glycosyl. These groups can be used to improve the biological properties of the antibody, such as increasing serum half-life.

Therefore, in certain embodiments, the antibody or antigen-binding fragment thereof of the present invention is labeled. In certain embodiments, the antibody or antigen-binding fragment thereof of the present invention is labeled with a detectable label, such as an enzyme, a radionuclide, a fluorescent dye, a luminescent substance (e.g., a chemiluminescent substance) or biotin. The detectable label of the present invention can be any substance that can be detected by fluorescent, spectroscopic, photochemical, biochemical, immunological, electrical, optical or chemical means. Such labels are well known in the art, and examples include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C or ³²P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivative (e.g., Cy7, Alexa 750)), luminescent substances (e.g., chemiluminescent substances such as acridinium ester compounds), magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above labels. In certain embodiments, such labels can be suitable for immunological detection (e.g., enzyme-linked immunosorbent assay, radioimmunoassay, fluorescent immunoassay, chemiluminescent immunoassay, etc.). In certain embodiments, the detectable label described above can be connected to the antibody or antigen-binding fragment thereof of the present invention through a linker of different lengths to reduce potential steric hindrance.

In another aspect, the present invention also provides a conjugate, which comprises the antibody or antigen-binding fragment thereof of the present invention and a therapeutic agent connected to the antibody or antigen-binding fragment thereof.

In certain embodiments, the conjugate is an antibody-drug conjugate (ADC). In certain preferred embodiments, the therapeutic agent is selected from the group consisting of cytotoxin or radioactive isotope. In the present invention, non-limiting examples of suitable therapeutic agents include antimetabolite, alkylating agent, DNA minor groove binder, DNA intercalator, DNA cross-linking agent, histone deacetylase inhibitor, nuclear export inhibitor, proteasome inhibitor, topoisomerase I or II inhibitor, heat shock protein inhibitor, tyrosine kinase inhibitor, antibiotic and anti-mitotic agent.

In certain embodiments, the antibody or antigen-binding fragment thereof of the present invention is optionally conjugated to the therapeutic agent through a linker. In certain embodiments, the antibody or antigen-binding fragment thereof of the present invention is conjugated to the therapeutic agent via a cleavable linker (e.g., a peptide linker, a disulfide or a hydrazone linker).

### Fusion protein

As described above, due to the small number of immune cells infiltrating in tumor tissues and/or the presence of a high proportion of immunosuppressive cells (e.g., Treg, MDSC), as well as the participation of other immunosuppressive pathways, the objective remission rate of anti-PD-1 antibody monotherapy for some tumor types is low.

To solve the above problems, the inventors of the present application further provide a fusion protein based on the PD-1 antibody with excellent performance to provide better anti-tumor activity.

In another aspect, the present invention also provides a fusion protein, which comprises the antibody or antigen-binding fragment thereof of the present invention and an additional biologically active polypeptide fused thereto.

In certain embodiments, the additional biologically active polypeptide is connected to the antibody or antigen-binding fragment thereof via a peptide linker. In certain embodiments, the peptide linker comprises one or more glycine (G) and/or one or more serine (S) and/or one or more alanine (A), such as a flexible peptide comprising (G₄S)ₙ or (G₄A)ₙ, wherein n=1, 2, 3 or 4. In certain exemplary embodiments, the peptide linker is (G₄A)₄G.

In certain embodiments, the additional biologically active polypeptide is an immunomodulator.

In certain embodiments, the immunomodulator is a TGF-β/TGF-βR pathway inhibitor.

Transforming growth factor-β (TGF-β) is a class of cytokines with multiple biological activities, secreted by tumor cells or immune cells, mainly including TGF-β1, TGF-β2 and TGF-β3. The TGF-β signaling pathway is an important factor in causing an immunosuppressive tumor microenvironment. The concentration of TGF-β in serum is negatively correlated with clinical outcomes. The main mechanisms comprise: (1) inhibiting the proliferation and activation of CD8+ T cells; (2) inhibiting the killing activity of NK cells; (3) inducing the production of immunosuppressive cells such as M2 macrophages, MDSCs and Tregs. In addition, TGF-β can also promote tumor cell invasion by inducing fibrosis and angiogenesis. Neutralizing TGF-β in the tumor microenvironment helps reduce tumor invasion, inhibit tumor growth and restore vascular normalization. At the same time, blocking TGF-β signals may enhance the clinical efficacy of immune checkpoint inhibitors.

Based on this, the inventors of the present application have developed a fusion protein comprising the PD-1 antibody of the present invention and a TGF-β/TGF-βR pathway inhibitor, which specifically binds to PD-1 and TGF-β and blocks the PD-L1/PD-1 and TGF-β/TGF-βR pathways, relieves T cell immunosuppression, better exerts anti-tumor activity, improves the objective response rate of tumor patients, and prolongs the survival of tumor patients.

In certain embodiments, the immunomodulator is a TGF-βRII extracellular domain. In certain embodiments, the TGF-βRII extracellular domain comprises a sequence as set forth in SEQ ID NO: 19.

In certain embodiments, the fusion protein comprises: a first peptide chain comprising a light chain of the antibody or antigen-binding fragment thereof: and a second peptide chain comprising a heavy chain of the antibody or antigen-binding fragment thereof, and an additional biologically active polypeptide.

In certain embodiments, the first peptide chain comprises a sequence as set forth in SEQ ID NO: 17, and/or the second peptide chain comprises a sequence as set forth in SEQ ID NO: 18.

### Polypeptide construct

When the antigen-binding fragment of the present invention is used, in order to increase its half-life, it can be connected to a biologically active polypeptide capable of increasing the half-life to form a construct. Therefore, the present invention also provides such a polypeptide construct.

In another aspect, the present invention also provides a polypeptide construct, which comprises the antibody or antigen-binding fragment thereof of the present invention and an immunoglobulin Fc domain.

In certain embodiments, the antibody or antigen-binding fragment thereof is an antigen-binding fragment. In certain embodiments, the antigen binding fragment is Fab, Fab', (Fab')₂, Fv, disulfide-linked Fv, scFv or diabody.

Herein, the Fc domain is also referred to as the Fc region, and refers to a portion of the heavy chain constant region comprising CH2 and CH3. In some embodiments, the Fc domain comprises a hinge, CH2 and CH3. When the Fc domain comprises a hinge, the hinge regulates the dimerization between two Fc-containing polypeptides. The Fc domain can be any antibody heavy chain constant region isotype. In some embodiments, the Fc domain is IgG1, IgG2, IgG3 or IgG4.

In certain embodiments, the Fc domain contained in the polypeptide construct of the present invention is a native Fc region, which comprises an amino acid sequence consistent with the amino acid sequence of the Fc region found in the nature. For example, the Fc domain can be a human IgG1 Fc region with native sequence, a human IgG2 Fc region with native sequence, a human IgG3 Fc region with native sequence or a human IgG4 Fc region with native sequence. The native Fc region can have effector functions. Exemplary "effector functions" include Fc receptor binding; Clq binding and complement-dependent cytotoxicity (CDC); antibody-dependent cell-mediated cytotoxicity (ADCC); phagocytosis; downregulation of cell surface receptor (e.g., B cell receptor); and B cell activation, etc. Functional changes can be produced by replacing at least one amino acid residue in the native Fc region with a different residue or chemical modification, for example, changing the affinity of the antibody for effector ligands (e.g., FcR or complement C1q), thereby changing the effector function (e.g., reducing or enhancing).

Therefore, in certain embodiments, the Fc domain contained in the polypeptide construct of the present invention can also be a variant Fc region, which can may comprise a mutation or chemical modification of one or more (e.g., 1-10, such as 1-5) amino acids as compared to the native Fc region so as to change one or more of the following properties of the antibody of the present invention: Fc receptor binding, antibody glycosylation, number of cysteine residues, effector cell function, or complement function, etc.

In certain embodiments, the Fc domain has reduced or eliminated ADCC activity, for example, comprises a LALA mutation (L234A, L235A).

In certain embodiments, the immunoglobulin Fc domain is an IgG Fc domain (e.g., an IgG1 Fc domain).

In certain exemplary embodiments, the immunoglobulin Fc domain comprises a sequence as set forth in SEQ ID NO: 22.

In certain embodiments, the immunoglobulin Fc domain is optionally connected to the antibody or antigen-binding fragment thereof (e.g., N-terminal and/or C-terminal) via a peptide linker. In certain embodiments, the peptide linker comprises one or more glycines (G) and/or one or more serines (S) and/or one or more alanines (A), for example, a flexible peptide comprising (G₄S)ₙ or (G₄A)ₙ, wherein n=1, 2, 3 or 4. In certain exemplary embodiments, the peptide linker is (G₄S)₂ or (G₄A)₂.

In certain embodiments, the polypeptide construct comprises the scFv described above and an immunoglobulin Fc domain. In certain embodiments, the immunoglobulin Fc domain is linked to the N-terminal of the scFv.

In certain exemplary embodiments, the polypeptide construct comprises a sequence as set forth in SEQ ID NO: 23 or 24.

### Preparation of antibody, fusion protein and polypeptide construct

The antibody of the present invention can be prepared by various methods known in the art, such as by genetic engineering recombinant technology. For example, a DNA molecule encoding the heavy chain and light chain genes of the antibody of the present invention is obtained by chemical synthesis or PCR amplification. The obtained DNA molecule is inserted into an expression vector and then transfected into a host cell. Then, the transfected host cell is cultured under specific conditions and the antibody of the present invention is expressed.

The antigen-binding fragments of the present invention can be obtained by hydrolyzing intact antibody molecules (see, Morimoto et al., J. Biochem. Biophys. Methods 24:107-117 (1992) and Brennan et al., Science 229:81 (1985)). In addition, these antigen-binding fragments can also be directly produced by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab or F(ab')₂ fragments can also be directly isolated from the culture medium of recombinant host cells. Ordinary technicians in this field are fully aware of other techniques for preparing these antigen-binding fragments.

Therefore, in another aspect, the present invention provides an isolated nucleic acid molecule, which comprises a nucleotide sequence encoding the antibody or antigen-binding fragment thereof of the present invention, or a heavy chain variable region and/or light chain variable region thereof. In certain embodiments, the isolated nucleic acid molecule encodes the antibody or antigen-binding fragment thereof of the present invention, or its heavy chain variable region and/or light chain variable region.

In another aspect, the present invention provides a vector (e.g., a cloning vector or an expression vector), which comprises the isolated nucleic acid molecule.

In another aspect, the present invention provides a host cell, which comprises the isolated nucleic acid molecule or vector. Such host cell includes, but is not limited to, prokaryotic cell such as *Escherichia coli* cell, and eukaryotic cell such as yeast cell, insect cell, plant cell and animal cell (e.g., mammalian cell, such as mouse cell, human cell, etc.).

In another aspect, a method for preparing the antibody or antigen-binding fragment thereof of the present invention is provided, which comprises: culturing the host cell under conditions that allow the expression of the antibody or antigen-binding fragment thereof, and recovering the antibody or antigen-binding fragment thereof from a culture of the cultured host cell.

In another aspect, the present invention also provides an isolated nucleic acid molecule, which comprises a nucleotide sequence encoding the fusion protein of the present invention. In certain embodiments, the isolated nucleic acid molecule encodes the fusion protein of the present invention. The present invention also provides a vector (e.g., a cloning vector or an expression vector), which comprises the isolated nucleic acid molecule. The present invention also provides a host cell, which comprises the isolated nucleic acid molecule or the vector. The present invention also provides a method for preparing the fusion protein of the present invention, which comprises: culturing the host cell under conditions that allow the expression of the fusion protein, and recovering the fusion protein from a culture of the cultured host cell.

In another aspect, the present invention also provides an isolated nucleic acid molecule, which comprises a nucleotide sequence encoding the polypeptide construct of the present invention. In certain embodiments, the isolated nucleic acid molecule encodes the polypeptide construct of the present invention. The present invention also provides a vector (e.g., a cloning vector or an expression vector), which comprises the isolated nucleic acid molecule. The present invention also provides a host cell, which comprises the isolated nucleic acid molecule or the vector. The present invention also provides a method for preparing the polypeptide construct of the present invention, which comprises culturing the host cell under conditions that allow the expression of the polypeptide construct, and recovering the polypeptide construct from a culture of the cultured host cell.

### Composition

In another aspect, the present invention also provides a composition comprising: (1) the antibody or antigen-binding fragment thereof or the polypeptide construct of the present invention; and (2) an immunomodulator.

In certain embodiments, the immunomodulator is a TGF-β/TGF-βR pathway inhibitor, such as the TGF-βRII extracellular domain.

In certain embodiments, the composition comprises: the antibody or antigen-binding fragment thereof of the present invention (e.g., a VH comprising a sequence as set forth in SEQ ID NO: 1, and a VL comprising a sequence as set forth in SEQ ID NO: 2), and a TGF-βRII extracellular domain.

In certain embodiments, the agents described in (1) and (2) are provided as separate components or as components of the same composition. Therefore, the agents described in (1) and (2) can be administered simultaneously, separately or sequentially.

### Pharmaceutical composition and therapeutic use

The antibody or antigen-binding fragment thereof of the present invention can be used in vitro or in vivo in a subject to inhibit and/or block intracellular signal transduction mediated by PD-1 binding to PD-L1, increase immune cell activity, enhance immune response, and prevent and/or treat a tumor or infection.

Therefore, in another aspect, the present invention provides a pharmaceutical composition, which comprises the antibody or antigen-binding fragment thereof, fusion protein, polypeptide construct or conjugate, or composition of the present invention, and a pharmaceutically acceptable carrier and/or excipient.

In certain embodiments, the pharmaceutical composition comprises a fusion protein comprising the antibody or antigen-binding fragment thereof of the present invention and a TGF-β/TGF-βR pathway inhibitor (e.g., TGF-βRII extracellular domain), which specifically binds to PD-1 and TGF-β at the same time and blocks PD-L1/PD-1 and TGF-β/TGF-βR pathways, relieves T cell immunosuppression, better exerts anti-tumor activity, improves the objective response rate of tumor patients, and prolongs the survival of tumor patients.

In certain embodiments, the pharmaceutical composition may also comprise an additional pharmaceutically active agent.

In certain embodiments, the additional pharmaceutically active agent is a medicament with anti-tumor activity, such as additional immune checkpoint inhibitor, oncolytic virus, chemotherapeutic agent, anti-angiogenic drug, antimetabolite, targeted tumor drug, immunostimulant, etc.

In certain embodiments, the additional pharmaceutically active agent is a medicament for treating an infection, such as antiviral agent, antifungal agent, antibacterial agent, immunostimulant, etc.

In certain embodiments, in the pharmaceutical composition, the antibody or antigen-binding fragment thereof, fusion protein, polypeptide construct or conjugate, or composition of the present invention and the additional pharmaceutically active agent are provided as separate components or as components of the same composition. Therefore, the antibody or antigen-binding fragment thereof, fusion protein, polypeptide construct or conjugate, or composition of the present invention and the additional pharmaceutically active agent can be administered simultaneously, separately or sequentially.

In certain exemplary embodiments, the medicament comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution and any combination thereof.

In another aspect, the present invention relates to a use of the antibody or antigen-binding fragment thereof, fusion protein, polypeptide construct or conjugate, or composition of the present invention in the preparation of a medicament, wherein the medicament is used for:
(1) increasing the activity of immune cells (e.g., T cells) in vitro or in a subject (e.g., a human);
(2) enhancing immune response (e.g., T cell-mediated immune response) in a subject (e.g., a human);
(3) treating a tumor in a subject (e.g., a human); or
(4) treating an infection in a subject (e.g., a human).

In certain embodiments, the medicament comprises a fusion protein comprising the antibody or antigen-binding fragment thereof of the present invention and a TGF-β/TGF-βR pathway inhibitor (e.g., an extracellular domain of TGF-βRII), which specifically binds to PD-1 and TGF-β at the same time and blocks PD-L1/PD-1 and TGF-β/TGF-βR pathways, relieves T cell immunosuppression, better exerts anti-tumor activity, improves the objective response rate of tumor patients, and prolongs the survival of tumor patients.

In another aspect, the present invention provides a method for increasing immune cell activity and/or enhancing immune response in a subject, the method comprising administering an effective amount of the antibody (or antigen-binding fragment thereof), fusion protein, polypeptide construct, conjugate, composition or pharmaceutical composition of the present invention to the subject in need thereof.

In certain embodiments, the immune response is a T cell-mediated immune response.

In certain embodiments, the method is used for preventing and/or treating a tumor. In such embodiments, the subject suffers from the tumor.

In certain embodiments, the method is used for preventing and/or treating an infection. In such embodiments, the subject suffers from the infection.

In certain embodiments, the immune cell is a T cell, such as a cytotoxic T cell (CTL), an antigen-specific T cell or a tumor-infiltrating T cell (TIL-T). In certain exemplary embodiments, the immune cell is a tumor-infiltrating lymphocyte, such as a tumor-infiltrating T cell.

In certain embodiments, a fusion protein comprising the antibody or antigen-binding fragment thereof of the present invention and a TGF-β/TGF-βR pathway inhibitor (e.g., TGF-βRII extracellular domain) is administered to the subject, which specifically binds to PD-1 and TGF-β at the same time and blocks PD-L1/PD-1 and TGF-β/TGF-βR pathways, relieves T cell immunosuppression, better exerts anti-tumor activity, improves the objective response rate of tumor patients, and prolongs the survival of tumor patients.

In another aspect, the present invention provides a method for preventing and/or treating a tumor in a subject (e.g., a human), comprising administering an effective amount of the antibody (or antigen-binding fragment thereof), fusion protein, polypeptide construct, conjugate, composition or pharmaceutical composition of the present invention to the subject in need thereof.

In certain embodiments, a fusion protein comprising the antibody or antigen-binding fragment thereof of the present invention and a TGF-β/TGF-βR pathway inhibitor (e.g., TGF-βRII extracellular domain) is administered to the subject, which specifically binds to PD-1 and TGF-β at the same time and blocks the PD-L1/PD-1 and TGF-β/TGF-βR pathways, relieves T cell immunosuppression, better exerts anti-tumor activity, improves the objective response rate of tumor patients, and prolongs the survival of tumor patients.

In certain embodiments, the antibody (or antigen-binding fragment thereof), fusion protein, polypeptide construct or conjugate, or composition of the present invention is used in combination with an additional drug with anti-tumor activity. The additional drug with anti-tumor activity can be administered before, simultaneously, or after the administration of the antibody (or antigen-binding fragment thereof), fusion protein, polypeptide construct or conjugate, or composition.

In certain embodiments, the antibody (or antigen-binding fragment thereof), fusion protein, polypeptide construct, conjugate, composition, or pharmaceutical composition of the present invention is administered in combination with an additional therapy. This additional therapy can be any therapy known for tumors, such as surgery, chemotherapy, radiotherapy, targeted therapy, immunotherapy, hormone therapy, gene therapy or palliative care. The additional therapy can be administered before, simultaneously or after the administration of the antibody (or antigen-binding fragment thereof), fusion protein, polypeptide construct, conjugate, composition or pharmaceutical composition of the present invention.

In another aspect, the present invention provides a method for preventing and/or treating an infection in a subject (e.g., a human), the method comprising administering an effective amount of the antibody (or antigen-binding fragment thereof), fusion protein, polypeptide construct, conjugate, composition or pharmaceutical composition of the present invention to the subject in need thereof.

In certain embodiments, the antibody (or antigen-binding fragment thereof), fusion protein, polypeptide construct or conjugate, or composition of the present invention is used in combination with an additional drug for treating infection. The additional drug for treating infection can be administered before, simultaneously or after the administration of the antibody (or antigen-binding fragment thereof), fusion protein, polypeptide construct or conjugate, or composition.

In certain embodiments of the use and method described above, the tumor is a tumor with microsatellite high instability (MSI-H) and/or mismatch repair deficiency (dMMR).

According to the MSI detection classification standard established by the National Cancer Institute (NCI) of the United States (Boland CR, et al. Cancer Res. 1998 Nov 15;58(22):5248-57.), for the five standard microsatellite loci, BAT26, BAT25, D2S123, D5S346, and D17S250, if the tumor tissue contains two or more unstable loci compared with normal tissue, it is judged as microsatellite high instability (MSI-H), that is, mismatch repair function defect (dMMR); if less than two or no loci are unstable, it is judged as microsatellite low instability (MSI-L) or microsatellite stable (MSS), that is, mismatch repair function is intact (pMMR).

Methods for detecting microsatellite high instability (MSI-H) or mismatch repair deficiency (dMMR) are known to those skilled in the art. For example, by detecting the length changes of the above five standard microsatellite loci in tumor tissue by PCR or second-generation sequencing, if two or more loci are unstable, it is judged as MSI-H. In addition, the expression of MLH1, MSH2, MSH6, and PSM2 in tumor tissues can also be detected by immunohistochemistry (ICH). If all four proteins are expressed positively, it is determined to be pMMR, and if any one of the proteins is expressed negatively, it is determined to be dMMR.

In certain embodiments of the above-mentioned uses and methods, the tumor is a solid tumor, such as melanoma (e.g., metastatic malignant melanoma), breast cancer, renal cancer (e.g., clear cell carcinoma), prostate cancer, bladder cancer, pancreatic cancer, lung cancer (e.g., non-small cell lung cancer), colon cancer, esophageal cancer, head and neck squamous cell carcinoma, liver cancer, ovarian cancer, cervical cancer, thyroid cancer, glioblastoma, and glioma.

In certain embodiments of the above-mentioned uses and methods, the tumor is a blood tumor, such as lymphoma or leukemia. In certain embodiments, the lymphoma is Hodgkin's lymphoma or non-Hodgkin's lymphoma. In certain embodiments, the non-Hodgkin's lymphoma is one or more of peripheral T-cell lymphoma, angioimmunoblastic T-cell lymphoma, NK/T-cell lymphoma (nasal type) with Epstein-Barr virus positivity, and B-cell non-Hodgkin's lymphoma.

In certain embodiments of the above-mentioned uses and methods, the infection refers to any infection caused by any pathogenic microorganisms such as viruses, bacteria, fungi, parasites, etc. In certain embodiments, the infection is selected from the group consisting of viral infection, bacterial infection, fungal infection, and parasitic infection.

In certain embodiments of the above-mentioned uses and methods, the subject is a mammal, such as a human.

The antibody or antigen-binding fragment thereof, fusion protein, polypeptide construct, conjugate, composition, and pharmaceutical composition of the present invention can be formulated into any dosage form known in the medical field, such as tablets, pills, suspensions, emulsions, solutions, gels, capsules, powders, granules, elixirs, lozenges, suppositories, injections (including injections, sterile powders for injection, and concentrated solutions for injection), inhalants, sprays, etc. The preferred dosage form depends on the intended mode of administration and therapeutic use. The pharmaceutical composition of the present invention should be sterile and stable under production and storage conditions. A preferred dosage form is an injection. Such an injection can be a sterile injection solution. For example, a sterile injection solution can be prepared by the following method: incorporating the necessary dose of the recombinant protein of the present invention into an appropriate solvent, and optionally, incorporating other desired ingredients (including but not limited to, pH adjuster, surfactant, adjuvant, ionic strength enhancer, isotonic agent, preservative, diluent, or any combination thereof), followed by filtration sterilization. In addition, the sterile injection solution can be prepared as a sterile lyophilized powder (e.g., by vacuum drying or freeze drying) for easy storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

In addition, the antibody or antigen-binding fragment thereof, fusion protein, polypeptide construct, and conjugate of the present invention may be present in a pharmaceutical composition in a unit dose form for ease of administration.

The antibody or antigen-binding fragment thereof, fusion protein, polypeptide construct, conjugate, composition, and pharmaceutical composition of the present invention may be administered by any suitable method known in the art, including, but not limited to, oral, buccal, sublingual, ocular, topical, parenteral, rectal, intrathecal, intracytoplasmic, inguinal, intravesical, topical (e.g., powders, ointments, or drops), or nasal routes. However, for many therapeutic uses, the preferred route/mode of administration is parenteral administration (e.g., intravenous injection or bolus, subcutaneous injection, intraperitoneal injection, intramuscular injection). The skilled person will appreciate that the route and/or mode of administration will vary depending on the intended purpose. In a preferred embodiment, the antibody or antigen-binding fragment thereof, fusion protein, polypeptide construct, conjugate, composition, or pharmaceutical composition of the present invention is administered by intravenous injection or bolus.

The pharmaceutical composition of the present invention may comprise a "therapeutically effective amount" or "prophylactically effective amount" of the antibody or antigen-binding fragment thereof, fusion protein, polypeptide construct, conjugate, or composition of the present invention. "Prophylactically effective amount" refers to an amount sufficient to prevent, arrest, or delay the occurrence of a disease. "Therapeutically effective amount" refers to an amount sufficient to cure or at least partially prevent a disease and complications thereof in a patient who already has the disease. The therapeutically effective amount of the antibody or antigen-binding fragment thereof, fusion protein, polypeptide construct or conjugate, or composition of the present invention may vary depending on the severity of the disease to be treated, the overall state of the patient's own immune system, the patient's general condition such as age, weight and gender, the mode of administration of the drug, and other treatments administered at the same time, etc.

### Kit and detection use

The antibody or antigen-binding fragment thereof of the present invention can specifically bind to PD-1, and can thus be used to detect the presence or level of PD-1 in a sample.

Therefore, in another aspect, the present invention provides a kit, which comprises the antibody or antigen-binding fragment thereof of the present invention In a preferred embodiment, the antibody or antigen-binding fragment thereof of the present invention carries a detectable label. In another preferred embodiment, the kit further comprises a second antibody that specifically recognizes the antibody or antigen-binding fragment thereof of the present invention. Preferably, the second antibody further comprises a detectable label.

In the present invention, the detectable label can be any substance that can be detected by fluorescent, spectroscopical, photochemical, biochemical, immunological, electrical, optical or chemical means. Particularly preferably, such label can be suitable for immunological detection (e.g., enzyme-linked immunosorbent assay, radioimmunoassay, fluorescence immunoassay, chemiluminescence immunoassay, etc.). Such labels are well known in the art and include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C or ³²P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750)), luminescent substances (e.g., chemiluminescent substances such as acridinium ester compounds), magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above-mentioned labels. In certain embodiments, the detectable labels as described above can be linked to the antibody or antigen-binding fragment thereof of the present invention via linkers of varying lengths to reduce potential steric hindrance.

In another aspect, the present invention provides a method for detecting the presence or level of PD-1 in a sample, which comprises a step of using the antibody or antigen-binding fragment thereof of the present invention. In a preferred embodiment, the antibody or antigen-binding fragment thereof of the present invention also carries a detectable label. In another preferred embodiment, the method further comprises detecting the antibody or antigen-binding fragment thereof of the present invention using a reagent with a detectable label. The method can be used for diagnostic purposes, or non-diagnostic purposes (e.g., the sample is a cell sample, not a sample from a patient). In certain embodiments, the PD-1 is human PD-1.

In another aspect, a use of the antibody or antigen-binding fragment thereof of the present invention in the manufacture of a kit for detecting the presence or level of PD-1 in a sample is provided. In certain embodiments, the PD-1 is human PD-1.

In certain embodiments, the sample is a cell sample (e.g., a tumor cell) or a tissue sample (e.g., a tumor tissue) from a subject (e.g., a mammal, such as a human).

### Definition of terms

In the present invention, unless otherwise specified, the scientific and technical terms used herein have the meanings commonly understood by those skilled in the art. Moreover, the cell culture, biochemistry, nucleic acid chemistry, immunology laboratory and other operation steps used herein are all routine steps widely used in the corresponding fields. At the same time, in order to better understand the present invention, the definitions and explanations of relevant terms are provided below.

As used herein, the term "antibody" refers to an immunoglobulin molecule generally composed of two pairs of polypeptide chains, each pair having a light chain (LC) and a heavy chain (HC). Antibody light chains can be classified as κ (kappa) and λ (lambda) light chains. Heavy chains can be classified as µ, δ, γ, α or ε, and the isotype of the antibody is defined as IgM, IgD, IgG, IgA and IgE, respectively. Within the light chain and heavy chain, the variable region and the constant region are connected by a "J" region of about 12 or more amino acids, and the heavy chain also contains a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin to host tissues or factors, including various cells of the immune system (e.g., effector cells) and the first component (C1q) of the classical complement system. The VH and VL regions can also be subdivided into highly variable regions (called complementary determining regions (CDRs)), interspersed with more conservative regions called framework regions (FRs). Each VH and VL consists of three CDRs and four FRs arranged from the amino terminus to the carboxyl terminus in the following order: FR1, CDR1, FR2, CDR2, FR3, CDR3, FR4. The variable regions (VH and VL) of each heavy chain/light chain pair form antigen binding sites, respectively. The distribution of amino acids in various regions or domains can follow the definitions of Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, the term "complementarity determining region" or "CDR" refers to those amino acid residues in a variable region of an antibody that are responsible for antigen binding. The precise boundaries of these amino acid residues can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, one skilled in the art will readily identify the CDRs defined by each numbering system. Moreover, the correspondence between different numbering systems is well known to those skilled in the art (for example, see Lefranc et al., Dev. Comparat. Immunol. 27:55-77, 2003).

In the present invention, the CDRs as contained in the antibody or antigen-binding fragment thereof of the present invention can be determined according to various numbering systems known in the art. In certain embodiments, the CDRs as contained in the antibody or antigen-binding fragment thereof of the present invention are determined by the numbering method described in the "Sequence analysis" section of Lu X, Nobrega RP, Lynaugh H, et al. Deamidation and isomerization liability analysis of 131 clinical-stage antibodies. MAbs. 2019 Jan;11(1):45-57. doi: 10.1080/19420862.2018.1548233. Page 11, and the entire contents of which are incorporated herein by reference.

As used herein, the term "framework region" or "FR" residues refers to those amino acid residues in a variable region of an antibody other than the CDR residues defined above.

The term "antibody" is not limited to any particular method of producing the antibody. For example, it includes recombinant antibodies, monoclonal antibodies and polyclonal antibodies. The antibody can be an antibody of different isotypes, for example, IgG (e.g., IgG1, IgG2, IgG3 or IgG4 subtype), IgA1, IgA2, IgD, IgE or IgM antibody.

As used herein, the term "antigen-binding fragment" of antibody refers to a polypeptide comprising a fragment of a full-length antibody that retains the ability to specifically bind to the same antigen bound by the full-length antibody and/or competes with the full-length antibody for specific binding to the antigen, which is also referred to as an "antigen-binding portion". See generally, Fundamental Immunology, Ch. 7 (Paul, W., ed., 2nd edition, Raven Press, N.Y. (1989), which is incorporated herein by reference in its entirety for all purposes. Antigen-binding fragments of antibodies can be produced by recombinant DNA techniques or by enzymatic or chemical cleavage of intact antibodies. Non-limiting examples of antigen-binding fragments include Fab, Fab', F(ab')₂, Fd, Fv, dAb and complementary determining region (CDR) fragments, single-chain antibodies (e.g., scFv), chimeric antibodies, diabodies, linear antibodies, nanobodies (technology from Domantis), domain antibodies (technology from Ablynx), probodies, and polypeptides that contain at least a portion of an antibody sufficient to confer specific antigen binding ability to the polypeptide. Engineered antibody variants are reviewed in Holliger et al., 2005; Nat Biotechnol, 23: 1126-1136.

As used herein, the term "full-length antibody" means an antibody consisting of two "full-length heavy chains" and two "full-length light chains", wherein, "full-length heavy chain" refers to a polypeptide chain that is composed of a heavy chain variable region (VH), a heavy chain constant region CH1 domain, a hinge region (HR), a heavy chain constant region CH2 domain, and a heavy chain constant region CH3 domain in the direction from N-terminal to C-terminal; and, when the full-length antibody is an IgE isotype, it optionally further comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. Two pairs of full-length antibody chains are linked together by disulfide bonds between CL and CH1 and between the HRs of two full-length heavy chains. The full-length antibody of the present invention may be from a single species, such as human; and it may also be a chimeric antibody or humanized antibody. The full-length antibody of the present invention comprises two antigen binding sites formed by VH and VL pairs, respectively, which specifically recognize/bind to the same antigen.

As used herein, the term "Fd fragment" refers to an antibody fragment consisting of VH and CH1 domains; the term "dAb fragment" refers to an antibody fragment consisting of VH domain (Ward et al., Nature 341:544 546 (1989)); the term "Fab fragment" refers to an antibody fragment consisting of VL, VH, CL and CH1 domains; the term "F(ab')₂ fragment" refers to an antibody fragment comprising two Fab fragments linked by a disulfide bridge at the hinge region; the term "Fab' fragment" refers to a fragment obtained after reduction of the disulfide bonds linking the two heavy chain fragments in F(ab')₂ fragment, consisting of a complete light chain and the Fd fragment of heavy chain (consisting of VH and CH1 domains).

As used herein, the term "Fv fragment" refers to an antibody fragment consisting of VL and VH domains of a single arm of an antibody. The Fv fragment is generally considered to be the smallest antibody fragment that can form a complete antigen binding site. It is generally believed that the six CDRs confer antigen binding specificity to an antibody. However, even a single variable region (e.g., an Fd fragment, which contains only three CDRs specific for an antigen) can recognize and bind to an antigen, although its affinity may be lower than that of the complete binding site.

As used herein, the term "Fc fragment" refers to an antibody fragment formed by disulfide bonding of the second and third constant regions of the first heavy chain of an antibody to the second and third constant regions of the second heavy chain. The Fc fragment of an antibody has a variety of different functions, but does not participate in antigen binding.

As used herein, the term "scFv" refers to a single polypeptide chain comprising VL and VH domains, wherein the VL and VH are connected by a linker. Such scFv molecules may have a general structure: NH₂-VL-linker-VH-COOH or NH₂-VH-linker-VL-COOH. Suitable prior art linkers consist of repeated GGGGS amino acid sequences or variants thereof. For example, a linker having an amino acid sequence (GGGGS)₄ may be used, but variants thereof may also be used (Holliger et al. (1993), Proc. Natl. Acad. Sci. USA 90: 6444-6448). In some cases, a disulfide bond may also exist between the VH and VL of scFv. As used herein, the term "di-scFv" refers to an antibody fragment formed by linking two scFvs.

As used herein, the term "diabody" means that its VH and VL domains are expressed on a single polypeptide chain, but a linker is too short to allow pairing between the two domains of the same chain, thereby forcing the domains to pair with the complementary domains of another chain and generate two antigen binding sites (see, for example, Holliger P. et al., Proc. Natl. Acad. Sci. USA 90:6444-6448 (1993), and Poljak R. J. et al., Structure 2:1121-1123 (1994)).

Each of the above antibody fragments retains the ability to specifically bind to the same antigen bound by the full-length antibody, and/or competes with the full-length antibody for specific binding to the antigen.

Antigen-binding fragments of antibody (e.g., the above-mentioned antibody fragments) can be obtained from a given antibody (e.g., the antibody provided by the present invention) using conventional techniques known to those skilled in the art (e.g., recombinant DNA technology or enzymatic or chemical cleavage methods), and the antigen-binding fragments of antibody can be screened for specificity in the same manner as for an intact antibody.

As used in herein, unless the context clearly indicates otherwise, when the term "antibody" is referred to, it includes not only an intact antibody but also an antigen-binding fragment of the antibody.

As used herein, the terms "monoclonal antibody", "McAb", and "mAb" have the same meaning and are used interchangeably, which refers to an antibody or a fragment of antibody from a group of highly homologous antibody molecules, that is, a group of completely identical antibody molecules except for natural mutations that may occur spontaneously. Monoclonal antibodies have high specificity for a single epitope on an antigen. Polyclonal antibodies are relative to monoclonal antibodies, which usually contain at least two or more different antibodies, which usually recognize different epitopes on an antigen. In addition, the modifier "monoclonal" only indicates that the antibody is characterized by being obtained from a highly homologous antibody group, and it cannot be understood that the antibody needs to be prepared by any specific method.

As used herein, the term "specific binding" refers to a non-random binding reaction between two molecules, such as a reaction between an antibody and an antigen to which it is directed. The strength or affinity of a specific binding interaction can be expressed by the equilibrium dissociation constant (K_{D}) of the interaction. In the present invention, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen. In certain embodiments, an antibody that specifically binds to an antigen (or an antibody that is specific to an antigen) refers to an antibody that binds to the antigen with an affinity (K_{D}) of less than about 10⁻⁹ M, such as less than about 10⁻⁹ M, 10⁻¹⁰ M, 10⁻¹¹ M or 10⁻¹² M or less. The specific binding properties between two molecules can be determined using methods known in the art, such as using surface plasmon resonance (SPR) in a BIACORE instrument.

As used herein, the term "vector" refers to a nucleic acid carrier into which a polynucleotide can be inserted. When a vector can express the protein encoded by the inserted polynucleotide, the vector is called an expression vector. The vector can be introduced into a host cell by transformation, transduction or transfection so that the genetic material elements it carries are expressed in the host cell. Vectors are well known to those skilled in the art, and include but are not limited to: plasmid; phagemid; cosmid; artificial chromosome, such as yeast artificial chromosome (YAC), bacterial artificial chromosome (BAC) or P1-derived artificial chromosome (PAC); bacteriophage such as lambda phage or M13 phage and animal virus. Animal viruses that can be used as vectors include but are not limited to retrovirus (including lentivirus), adenovirus, adeno-associated virus, herpes virus (e.g., herpes simplex virus), poxvirus, baculovirus, papillomavirus, papovavirus (e.g., SV40). A vector may contain a variety of elements for controlling expression, including but not limited to promoter sequence, transcription start sequence, enhancer sequence, selection element and reporter gene. In addition, the vector may also contain a replication origin.

As used herein, the term "host cell" refers to a cell that can be used to introduce a vector, including but not limited to prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "identity" is used to refer to the matching of sequences between two polypeptides or between two nucleic acids. When a position in both compared sequences is occupied by the same base or amino acid monomer subunit (for example, a position in each of the two DNA molecules is occupied by adenine, or a position in each of the two polypeptides is occupied by lysine), then the molecules are identical at that position. "Percent identity" between two sequences is a function of the number of matching positions shared by the two sequences divided by the number of positions compared x 100. For example, if 6 out of 10 positions of two sequences match, then the two sequences have 60% identity. For example, the DNA sequences CTGACT and CAGGTT have an identity of 50% (3 out of a total of 6 positions match). Typically, two sequences are compared when aligned to produce maximum identity. Such an alignment can be achieved by using, for example, the method of Needleman et al. (1970) J. Mol. Biol. 48: 443-453, which can be conveniently performed by a computer program such as the Align program (DNAstar, Inc.). The percent identity between two amino acid sequences can also be determined using the algorithm of E. Meyers and W. Miller (Comput. Appl Biosci., 4:11-17 (1988)) which has been incorporated into the ALIGN program (version 2.0), using a PAM120 weight residue table, a gap length penalty of 12, and a gap penalty of 4. In addition, the percent identity between two amino acid sequences can be determined using the algorithm of Needleman and Wunsch (J MoI Biol. 48:444-453 (1970)), which has been incorporated into the GAP program of the GCG software package (available at www.gcg.com), using a Blossum 62 matrix or a PAM250 matrix, a gap weight of 16, 14, 12, 10, 8, 6, or 4 and a length weight of 1, 2, 3, 4, 5, or 6.

As used herein, the term "conservative substitution" refers to an amino acid substitution that does not adversely affect or change the expected properties of the protein/polypeptide comprising the amino acid sequence. For example, a conservative substitution can be introduced by standard techniques known in the art such as site-directed mutagenesis and PCR-mediated mutagenesis. Conservative amino acid substitutions include substitutions of amino acid residues with amino acid residues having similar side chains, such as substitutions with residues physically or functionally similar to the corresponding amino acid residues (e.g., having similar size, shape, charge, chemical properties, including the ability to form covalent bond or hydrogen bond, etc.). Families of amino acid residues with similar side chains have been defined in the art. These families comprise amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, it is preferred to substitute a corresponding amino acid residue with another amino acid residue from the same side chain family. Methods for identifying conservative substitutions of amino acids are well known in the art (see, e.g., Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes, but is not limited to: pH regulator, surfactant, adjuvant, ionic strength enhancer, diluent, agent for maintaining osmotic pressure, agent for delaying absorption, preservative. For example, the pH regulator includes, but is not limited to, phosphate buffer. The surfactant includes, but is not limited to, cationic, anionic or nonionic surfactant, such as Tween-80. The ionic strength enhancer includes, but is not limited to, sodium chloride. The preservative includes, but is not limited to, various antibacterial and antifungal agent, such as paraben, chlorobutanol, phenol, sorbic acid, and the like. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), etc. The preservative includes, but is not limited to, various antibacterial and antifungal agent, such as thimerosal, 2-phenoxyethanol, paraben, chlorobutanol, phenol, sorbic acid, etc. Stabilizers have the meanings generally understood by those skilled in the art, which can stabilize the desired activity of the active ingredient in the drug, including but not limited to sodium glutamate, gelatin, SPGA, sugars (e.g., sorbitol, mannitol, starch, sucrose, lactose, dextran, or glucose), amino acids (e.g., glutamic acid, glycine), proteins (e.g., dried whey, albumin or casein) or their degradation products (e.g., lactalbumin hydrolysate), etc. In certain exemplary embodiments, the pharmaceutically acceptable carrier or excipient comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solutions), Ringer's solution, and any combination thereof.

As used herein, the term "prevention" refers to a method performed to prevent or delay the occurrence of a disease or condition or symptom (e.g., a tumor or infection) in a subject. As used herein, the term "treatment" refers to a method performed to obtain a beneficial or desired clinical result. For the purposes of the present invention, beneficial or desired clinical results include, but are not limited to, alleviation of symptom, reduction of the extent of the disease, stabilization (i.e., no longer worsening) of the state of the disease, delay or slowing of the progression of the disease, improvement or alleviation of the state of the disease, and relief of symptom (whether partial or complete), whether detectable or undetectable. In addition, "treatment" may also refer to prolonging survival compared to the expected survival if no treatment is received.

As used herein, the term "subject" refers to a mammal, such as a primate mammal, such as a human. In certain embodiments, the subject (e.g., a human) suffers from a tumor or infection, or is at risk of suffering from the above-mentioned disease.

As used herein, the term "effective amount" refers to an amount sufficient to obtain or at least partially obtain the desired effect. For example, an effective amount for preventing a disease (e.g., a tumor or infection) refers to an amount sufficient to prevent, arrest, or delay the occurrence of a disease (e.g., a tumor or infection); an effective amount for treating a disease refers to an amount sufficient to cure or at least partially prevent the disease and its complications in a patient who has already suffered from the disease. Determining such an effective amount is entirely within the capabilities of those skilled in the art. For example, an effective amount for therapeutic use will depend on the severity of the disease to be treated, the overall state of the patient's own immune system, the patient's general condition such as age, weight and gender, the mode of administration of the drug, and other treatments administered simultaneously, etc.

### Beneficial effects of the invention

The antibody of the present invention can not only specifically recognize/bind to PD-1, block the binding of PD-1 to PD-L1, but also enhance immune cell activity in vitro/in vivo and stimulate immune responses. Therefore, the antibody of the present invention has the potential to be used for the prevention and/or treatment of tumors or infections. In addition, the present invention also provides a fusion protein comprising the antibody of the present invention and a TGF-β/TGF-βR pathway inhibitor, which specifically binds to PD-1 and TGF-β at the same time and blocks the PD-L1/PD-1 and TGF-β/TGF-βR pathways, relieves T cell immunosuppression, better exerts anti-tumor activity, improves the objective response rate of tumor patients, and prolongs the survival of tumor patients.

The embodiments of the present invention will be described in detail below in conjunction with the accompanying drawings and examples, but those skilled in the art will understand that the following drawings and examples are only used to illustrate the present invention, rather than to limit the scope of the present invention. According to the following detailed description of the accompanying drawings and preferred embodiments, the various objects and advantages of the present invention will become apparent to those skilled in the art.

### Brief Description of the Drawings

Figure 1A shows a binding curve of anti-PD-1 antibody/scFv to human PD-1 overexpressed on CHO cells.
Figure 1B shows a binding curve of anti-PD-1 antibody/scFv to cynomolgus monkey PD-1 overexpressed on CHO cells.
Figure 2 shows a curve of anti-PD-1 antibody/scFv blocking the binding of human PD-L1 to human PD-1 overexpressed on CHO cells.
Figure 3 shows a binding curve of anti-PD-1 antibody/scFv to PD-1 on activated human primary T cells.
Figure 4 shows a curve of anti-PD-1 antibody/scFv blocking PD-L1/PD-1 luciferase reporter gene.
Figure 5 shows a bar graph of stimulating IL-2 secretion by anti-PD-1 antibody/scFv in a mixed lymphocyte assay.
Figure 6 shows a pharmacodynamic graph of anti-PD-1 antibody in h-PD-1 KI mice bearing h-PD-L1 KI MC38 tumor model.
Figure 7 shows a pharmacodynamic graph of anti-PD-1 antibody in B-NDG B2M KO plus mice incubated with A375 tumor cells model.
Figure 8 shows a schematic diagram of the molecular structure of anti-PD-1/TGF-β fusion protein 54872-TGF-βRII.
Figure 9A shows a binding curve of anti-PD-1/TGF-β fusion protein 54872-TGF-βRII to human PD-1 overexpressed on CHO cells.
Figure 9B shows a binding curve of anti-PD-1/TGF-β fusion protein 54872-TGF-βRII to cynomolgus monkey PD-1 overexpressed on CHO cells.
Figure 10 shows a curve of anti-PD-1/TGF-β fusion protein 54872-TGF-βRII blocking the binding of human PD-L1 to human PD-1 overexpressed on CHO cells.
Figure 11 shows a curve of anti-PD-1/TGF-β fusion protein 54872-TGF-βRII blocking PD-L1/PD-1 luciferase reporter gene.
Figures 12A to 12C show binding curves of anti-PD-1/TGF-β fusion protein 54872-TGF-βRII to human TGF-β1, TGF-β2, TGF-β3.
Figure 13 shows a curve of anti-PD-1/TGF-β fusion protein 54872-TGF-βRII blocking TGF-β1/SMAD signaling pathway.
Figure 14A shows an ELISA level co-binding curve of 54872-TGF-βRII molecule to human PD-1 protein and anti-TGF-βRII antibody (human PD-1 protein coated on the plate, biotin labeled anti-TGF-βRII antibody as detection signal).
Figure 14B shows an ELISA level co-binding curve of 54872-TGF-βRII molecule to TGF-β1 and human PD-1 protein (TGF-β1 coated on the plate, biotin labeled PD-1 protein as detection signal).
Figure 15 shows a curve of binding of 54872-TGF-βRII molecule to human PD-1 overexpressed on CHO cells, in which the binding is not affected by the presence of TGF-β1.
Figure 16 shows a curve of 54872-TGF-βRII molecule blocking the binding of human PD-L1 to human PD-1 overexpressed on CHO cells that is not affected by the binding or not binding of TGF-β1.
Figures 17A to 17B show bar graphs of release of IL-2 and IFN-γ activated by 54872-TGF-βRII molecule in a mixed lymphocyte assay.
Figure 18 shows anti-tumor efficacy of 54872-TGF-βRII molecule in B-NDG B2M KO mice incubated with A375 tumor cells model.
Figure 19 shows a curve of serum concentration over time of 54872-TGF-βRII molecule in mice.

### Sequence information

The information of the sequence involved in the present invention is provided in Table 1 below.

**Table 1: Description of sequence**

| SEQ ID NO | Description | Sequence information |
|---|---|---|
| 1 | ADI-54872 VH | QVQLVQSGAEVKKPGASVKVSCKASGYTFTEYYIYWVRQ APGQGLEWIGGINPSNGGTNFNEKFKPRVTMTVDTSTSTAY MELSSLRSEDTAVYYCTVRDFRFDKGFKYWGQGTLVTVSS |
| 2 | ADI-54872 VL | EIVLTQSPATLSLSPGERATLSCRASKSVSTSGLNYVHWYQ RKPGQAPRLLIYLGSYLDSGVPARFSGSGSGTDFTLTISSLEP EDFAVYYCQQSWELPLTFGGGTKVEIK |
| 3 | ADI-54872-1 VH | QVQLVQSGAEVKKPGASVKVSCKASGYTFTEYYIYWVRQ APGQCLEWIGGINPSNGGTNFNEKFKPRVTMTVDTSTSTAY MELSSLRSEDTAVYYCTVRDFRFDKGFKYWGQGTLVTVSS |
| 4 | ADI-54872-1 VL | EIVLTQSPATLSLSPGERATLSCRASKSVSTSGLNYVHWYQ RKPGQAPRLLIYLGSYLDSGVPARFSGSGSGTDFTLTISSLEP EDFAVYYCQQSWELPLTFGCGTKVEIK |
| 5 | ADI-63628 VH | QVQLVQSGAEVKKPGASVKVSCKASGFTFTEYYIYWVRQ APGQCLEWIGGINPSNGGTNFNEKFKPRVTMTVDTSTSTAY MELSSLRSEDTAVYYCTVRDFRFDKGFKYWGQGTLVTVSS |
| 6 | ADI-63628 VL | EIVLTQSPATLSLSPGERATLSCRASKSVSTSGLNYVHWYQ RKPGQAPRLLIYLGSYRDSGVPARFSGSGSGTDFTLTISSLE PEDFAVYYCQQSWELPLTFGCGTKVEIK |
| 7 | ADI-54872/ADI-54872-1 | YTFTEYYIY |
| | HCDR-1 | |
| 8 | ADI-54872/ADI-54872-1/ADI-63628 | GINPSNGGTNFNEKFKP |
| | HCDR-2 | |
| 9 | ADI-54872/ADI-54872-1/ADI-63628 | TVRDFRFDKGFKY |
| | HCDR-3 | |
| 10 | ADI-54872/ADI-54872-1/ADI-63628 | RASKSVSTSGLNYVH |
| | LCDR-1 | |
| 11 | ADI-54872/ADI-54872-1 | LGSYLDS |
| | LCDR-2 | |
| 12 | ADI-54872/ADI-54872-1/ADI-63628 | QQSWELPLT |
| | LCDR-3 | |
| 13 | ADI-63628 | FTFTEYYIY |
| | HCDR-1 | |
| 14 | ADI-63628 | LGSYRDS |
| | LCDR-2 | |
| 15 | Human IgG1 LALA heavy chain constant region | ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALPAPIEKTISKAKGQPREPQVYTLPPSRDELTK NQVSLTCLVKGFYPSDIAVEWESNGQPENNYKTTPPVLDS DGSFFLYSKLTVDKSRWQQGNVFSCSVMHEALHNHYTQK SLSLSPG |
| 16 | Light chain constant region | |
| 17 | 54872-TGF-βRII heavy chain | QVQLVQSGAEVKKPGASVKVSCKASGYTFTEYYIYWVRQ APGQGLEWIGGINPSNGGTNFNEKFKPRVTMTVDTSTSTAY MELSSLRSEDTAVYYCTVRDFRFDKGFKYWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPEAAGGPSV FLFPPKPKDTLMISRTPEVTCVVVDVSHEDPEVKFNWYVD GVEVHNAKTKPREEQYNSTYRVVSVLTVLHQDWLNGKEY KCKVSNKALPAPIEKTISKAKGQPREPQVYTLPPSRDELTK NQVSLTCLVKGFYPSDIAVEWESNGQPENNYKTTPPVLDS DGSFFLYSKLTVDKSRWQQGNVFSCSVMHEALHNHYTQK SLSLSPGGGGGAGGGGAGGGGAGGGGAGIPPHVQKSVNN DMIVTDNNGAVKFPQLCKFCDVRFSTCDNQKSCMSNCSIT SICEKPQEVCVAVWRKNDENITLETVCHDPKLPYHDFILED AASPKCIMKEKKKPGETFFMCSCSSDECNDNIIFSEEYNTSN PD |
| 18 | 54872-TGF-βRII light chain | EIVLTQSPATLSLSPGERATLSCRASKSVSTSGLNYVHWYQ RKPGQAPRLLIYLGSYLDSGVPARFSGSGSGTDFTLTISSLEP EDFAVYYCQQSWELPLTFGGGTKVEIKRTVAAPSVFIFPPS DEQLKSGTASVVCLLNNFYPREAKVQWKVDNALQSGNSQ |
| | | ESVTEQDSKDSTYSLSSTLTLSKADYEKHKVYACEVTHQG LSSPVTKSFNRGEC |
| 19 | TGF-βRII extracellular region | IPPHVQKSVNNDMIVTDNNGAVKFPQLCKFCDVRFSTCDN QKSCMSNCSITSICEKPQEVCVAVWRKNDENITLETVCHDP KLPYHDFILEDAASPKCIMKEKKKPGETFFMCSCSSDECND NIIFSEEYNTSNPD |
| 20 | Human PD-1 ECD | FLDSPDRPWNPPTFSPALLVVTEGDNATFTCSFSNTSESFVL NWYRMSPSNQTDKLAAFPEDRSQPGQDCRFRVTQLPNGR DFHMSVVRARRNDSGTYLCGAISLAPKAQIKESLRAELRVT ERRAEVPTAHPSPSPRPAGQFQTLV |
| 21 | Cynomolgus monkey PD-1 ECD | MQIPQAPWPVVWAVLQLGWRPGWFLESPDRPWNAPTFSP ALLLVTEGDNATFTCSFSNASESFVLNWYRMSPSNQTDKL AAFPEDRSQPGQDCRFRVTRLPNGRDFHMSVVRARRNDSG TYLCGAISLAPKAQIKESLRAELRVTERRAEVPTAHPSPSPR PAGQ |
| 22 | IgG1 CH2/CH3-LALA constant region sequence (Fc) | DKTHTCPPCPAPELLGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSRDELTKNQVSLTCLVKGFYPSDIAVEWE SNGQPENNYKTTPPVLDSDGSFFLYSKLTVDKSRWQQGNV FSCSVMHEALHNHYTQKSLSLSPG |
| 23 | ADI-54872-1 full-length sequence | DKTHTCPPCPAPELLGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSRDELTKNQVSLTCLVKGFYPSDIAVEWE SNGQPENNYKTTPPVLDSDGSFFLYSKLTVDKSRWQQGNV FSCSVMHEALHNHYTQKSLSLSPGGGGGSGGGGSQVQLVQ SGAEVKKPGASVKVSCKASGYTFTEYYIYWVRQAPGQCLE WIGGINPSNGGTNFNEKFKPRVTMTVDTSTSTAYMELSSLR SEDTAVYYCTVRDFRFDKGFKYWGQGTLVTVSSGGGGSG GGGSGGGGSGGGGSEIVLTQSPATLSLSPGERATLSCRASK SVSTSGLNYVHWYQRKPGQAPRLLIYLGSYLDSGVPARFS GSGSGTDFTLTISSLEPEDFAVYYCQQSWELPLTFGCGTKV EIK |
| 24 | ADI-63628 full-length sequence | DKTHTCPPCPAPELLGGPSVFLFPPKPKDTLMISRTPEVTCV VVDVSHEDPEVKFNWYVDGVEVHNAKTKPREEQYNSTYR VVSVLTVLHQDWLNGKEYKCKVSNKALPAPIEKTISKAKG QPREPQVYTLPPSRDELTKNQVSLTCLVKGFYPSDIAVEWE SNGQPENNYKTTPPVLDSDGSFFLYSKLTVDKSRWQQGNV FSCSVMHEALHNHYTQKSLSLSPGGGGGAGGGGAQVQLV QSGAEVKKPGASVKVSCKASGFTFTEYYIYWVRQAPGQCL EWIGGINPSNGGTNFNEKFKPRVTMTVDTSTSTAYMELSSL RSEDTAVYYCTVRDFRFDKGFKYWGQGTLVTVSSGGGGS GGGGSGGGGSGGGGSEIVLTQSPATLSLSPGERATLSCRAS KSVSTSGLNYVHWYQRKPGQAPRLLIYLGSYRDSGVPARF SGSGSGTDFTLTISSLEPEDFAVYYCQQSWELPLTFGCGTK VEIK |

### Specific Models for Carrying Out the Invention

The present invention is now described with reference to the following examples which are intended to illustrate the present invention (but not to limit the present invention). Unless otherwise specified, the molecular biology experimental methods and immunoassays used in the present invention were performed basically based on the methods described in J. Sambrook et al., Molecular Cloning: A Laboratory Manual, 2nd edition, Cold Spring Harbor Laboratory Press, 1989, and F. M. Ausubel et al., Compiled Molecular Biology Laboratory Manual, 3rd edition, John Wiley & Sons, Inc., 1995; the use of restriction endonucleases was in accordance with the conditions recommended by the product manufacturer. Those skilled in the art will appreciate that the examples describe the present invention by way of example and are not intended to limit the scope of protection claimed by the present invention.

The control antibody Pembrolizumab involved in the present invention: anti-PD-1 monoclonal antibody, trade name Keytruda, was an original drug produced by Merck.

### Example 1: Screening, sequence design, expression and purification of antibody

### 1. Animal immunization and single cell sorting

The mouse IgG1 Fc fusion protein of PD-1 extracellular segment (Genbank sequence number: AY238517) was mixed with Ribi adjuvant (sigma), and subcutaneously immunized into 6- to 8-week-old BALB/c mice at a dose of 10 µg/mouse. The PD-1 extracellular segment-his fusion protein was taken, and intraperitoneally injected at a dose of 10 µg/mouse for booster immunization. The mice were euthanized, the spleen and lymph nodes were taken, placed on a cell screen, gently ground, and rinsed with PBS to obtain a single cell suspension. The cells were co-incubated with biotin-labeled PD-1 protein and biotin-binding fluorescent secondary antibody, washed, resuspended in FACS buffer, and sorted by flow cytometry. The single cells were sorted into a 96-well PCR plate (BioRad). Each well was added with 20 µL of lysis buffer (5 µL of 5X single-stranded cDNA buffer (Invitrogen), 0.625 µL of NP-40 (New England Biolabs), 0.25 µL of RNaseOUT (Invitrogen), 1.25 µL of dithiothreitol (Invitrogen), and 12.6 µL of double distilled water). After sorting, the plate was immediately transferred to -80°C for storage.

### 2. Cloning and amplification of variable region sequences of single B cell antibody

According to the previous report (Tiller et al. 2009 J Immunol Methods 350:183-93), specific IgG and IgM specific mixed primers were used, and the cDNA sequences encoding the heavy chain variable region and light chain variable region of the antibody were amplified from the B cell lysate by reverse transcription PCR and nested PCR. 10 µL of the heavy and light chain variable region sequences amplified by PCR and 200 ng of yeast expression plasmids digested with enzymes were co-transfected into yeast cells. The transfected single yeast cells were used to perform subsequent enrichment, amplification, antibody expression, screening, sequencing, etc.

### 3. Humanization of anti-PD-1 antibody

For example, to reduce the potential immunogenicity of mouse antibody, the antibody was humanized. Generally speaking, the humanized antibody contained the CDR region of non-human antibody and the framework region and constant region of human antibody. In some cases, some amino acids in the framework region of human antibody were also reversely mutated to better maintain the activity of the antibody. Humanization was performed according to previous reports (Imagro et al. Front. Biosci. 13:1619-1633 (2008); Riechmann et al. Nature 332:323-329 (1988); Queen et al. Proc. Nat'l Acad. Sci. USA 86:10029-10033 (1989); U.S. Pat. Nos. 5,821,337, 7,527,791, 6,982,321, 7,087,409; Kashmiri et al. Methods 36:25-34 (2005); Padlan, Mol. Immunol. 28:489-498 (1991); Dall'Acqua et al. Methods 36:43-60 (2005)). Methods for selecting human antibody framework regions include, but are not limited to, the "optimal selection method" (Sims et al. J. Immunol. 151:2296 (1993)). The antibody framework regions were derived from the sequence comparison method of heavy and light chain variable region libraries (Carter et al. Proc. Natl. Acad. Sci. USA, 89:4285). (1992) etc. The antibody humanization modification of the present invention was based on the humanized antibody sequence library computational biology selection and the most suitable human antibody framework region of the mouse antibody, and the mouse antibody CDR region was grafted onto the human framework region. For each humanized parent antibody, 3 to 4 most suitable human antibody heavy chain variable regions and 3 to 4 most suitable human antibody light chain variable regions were selected and constructed according to the above method. The antibody was expressed by transfection into yeast, and subjected to human/monkey PD-1 protein binding screening.

### 4. Antibody engineering optimization

Further engineering optimization of monoclonal antibody/scFv after humanization was carried out as follows: random point mutation was performed on the antibody heavy chain variable region CDRs 1, 2, 3 and the original light chain was paired and transfected into yeast, and a library was constructed and screened to obtain a yeast population with improved affinity; and a plasmid library with improved heavy chain affinity was obtained by extraction. Random point mutation was performed on the antibody light chain variable region CDRs 1, 2, 3 and the original heavy chain was paired and transfected into yeast, and a library was constructed and screened to obtain a yeast population with improved affinity; and a plasmid library with improved light chain affinity was obtained by extraction. The plasmid library with improved heavy chain affinity and the plasmid library with improved light chain affinity were randomly mixed and transfected into yeast, and library construction and screening were performed to obtain a final yeast monoclonal clone with improved affinity, which was sequenced to obtain an antibody sequence with improved affinity.

The anti-PD-1 antibody ADI-54872 was finally obtained through screening and modification by the above method, and its derivative (anti-PD-1 scFv molecule), named ADI-54872-1, was further obtained based on the variant region sequence, and ADI-54872-1 was further engineered to obtain its derivative ADI-63628. The full-length sequences of ADI-54872-1 and ADI-63628 were set forth in SEQ ID NO: 23 and 24, respectively, which contained IgG1 CH2/CH3 constant region sequence (SEQ ID NO: 22) and scFv sequence (VH-linker-VL). The CDR and variable region sequences of the above anti-PD-1 antibody and scFv derivatives thereof were shown in the following table.

**Table 2: Antibody CDR and variable region sequences**

| | SEQ ID NO: | | | | | | | |
|---|---|---|---|---|---|---|---|---|
| Ab | VH | VL | VH CDR1 | VH CDR2 | VH CDR3 | VL CDR1 | VL CDR2 | VL CDR3 |
| ADI-54872 | 1 | 2 | 7 | 8 | 9 | 10 | 11 | 12 |
| ADI-54872-1 | 3 | 4 | 7 | 8 | 9 | 10 | 11 | 12 |
| ADI-63628 | 5 | 6 | 13 | 8 | 9 | 10 | 14 | 12 |

After the amino acid sequence of the heavy chain variable region and the amino acid of the heavy chain constant region (SEQ ID NO: 15) of ADI-54872 were connected, and the amino acid sequence of the light chain variable region and the amino acid sequence of the light chain constant region (SEQ ID NO: 16) of ADI-54872 were connected, they were cloned into pcDNA3.1 expression vector, respectively, and the antibody ADI-54872 was transiently expressed and purified via the HEK293 expression system. The amino acid sequence of ADI-54872-1 (SEQ ID NO: 23) and the amino acid sequence of ADI-63628 (SEQ ID NO: 24) were cloned into pcDNA3.1 expression vector, respectively, and the corresponding scFv derivatives ADI-54872-1 and ADI-63628 were transiently expressed and purified via the HEK293 expression system. The specific operation was follows: the pcDNA3.1 vector carrying the antibody heavy chain and/or light chain was transferred into HEK293 cells by chemical transfection, and cultured at 37°C and 8% CO₂ for 7 days. The cytochylema was collected and centrifuged at 13000rpm for 20 minutes. The supernatant was taken, and purified by Protein A, and the antibody purity was detected by SEC, while the endotoxin content was controlled.

### Example 2: Detection of antibody affinity

The binding dissociation constant (K_{D}) values of the antibody obtained in Example 1 binding to human and cynomolgus monkey PD-1 were determined by biofilm optical interference technology (ForteBio). Fortebio affinity determination was carried out according to the existing method (Este, P et al. High throughput solution-based measurement of antibody-antigen affinity and epitope binning. Mabs, 2013.5(2):p.270-8), and the extracellular amino acid sequences of human and cynomolgus monkey PD-1 were as set forth in SEQ ID NO:20 and 21, respectively.

Measurement of monovalent affinity of intact antibody (full-length IgG) or anti-PD-1 Fc-scFv protein to human and cynomolgus monkey PD-1-his protein: The sensor was equilibrated offline in the assay buffer for 20 minutes, then tested online for 120 s to establish a baseline, and the intact PD-1 antibody or Fc-scFv was loaded onto the AHQ sensor to a thickness of 1 nm for affinity detection. The sensor loaded with antibody was exposed to 100 nM PD-1-his antigen until the plateau phase, and then the sensor was transferred to the assay buffer for at least 2 minutes for dissociation rate measurement. The kinetic analysis was performed using a 1:1 binding model.

The results were shown in the table below. ADI-54872, ADI-54872-1, and ADI-63628 had better monovalent affinity for binding to human and cynomolgus monkey PD-1 proteins than the control antibody pembrolizumab.

**Table 3: K_{D} values of antibodies**

| Clone | IgG binding to Human PD-1-his K_{D} (M) | IgG or Fc-scFv binding to Cyno PD-1-his K_{D} (M) |
|---|---|---|
| ADI-54872 | 4.53E-10 | 2.01E-10 |
| ADI-54872-1 | 1.17E-09 | 1.05E-09 |
| ADI-63628 | 3.90E-10 | 2.38E-10 |
| Pembrolizumab | 9.68E-09 | 2.98E-09 |

### Example 3: Binding activity of anti-PD-1 antibody/scFv to CHO cells overexpressing human/cynomolgus monkey PD-1

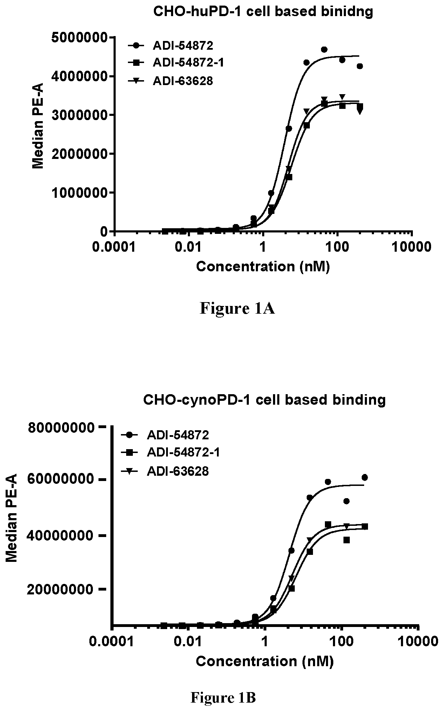

By transfecting human PD-1 (Uniprot: Q15116), cynomolgus monkey PD-1 (Uniprot: B0LAJ3) cDNA pCHO1.0 vector (purchased from Invitrogen) into CHO-S cellsand following with pressure screening, CHO-S cells overexpressing human PD-1 (CHO-huPD-1 cells) and CHO-S cells overexpressing cynomolgus monkey PD-1 (CHO-cynoPD-1 cells) were produced. The overexpressed cells after expansion culture were adjusted to the appropriate cell density and added to a 96-well flow plate, centrifuged and then added with gradiently diluted samples, and incubated at 4°C for 30 minutes. Washing was performed twice with PBS, fluorescent secondary antibody correspondingly diluted to the appropriate concentration was added, incubated at 4°C for 30 minutes, and washed twice with PBS. The cells were resuspended in PBS, and detected on a CytoFlex flow cytometer, and the corresponding MFI was calculated. Graphpad software was used for graphical analysis to obtain EC₅₀ values. The results were shown in Table 4 and Figures 1A and 1B: ADI-54872, ADI-54872-1, and ADI-63628 had good binding activity to CHO cells overexpressing human PD-1 and cynomolgus monkey PD-1.

**Table 4: Summary of binding and blocking activities of anti-PD-1 antibodies/scFv at overexpression cell level**

| Antibody | EC₅₀ (nM) of binding to CHO cells overexpressing human PD-1 | EC₅₀ (nM) of binding to CHO cells overexpressing cynomolgus monkey PD-1 | IC₅₀ (nM) of blocking human PD-L1 binding based on CHO cells overexpressing human PD-1 |
|---|---|---|---|
| ADI-54872 | 3.785 | 4.319 | 3.737 |
| ADI-54872-1 | 5.737 | 6.452 | 7.137 |
| ADI-63628 | 4.817 | 5.184 | 6.107 |

### Example 4: Activity of anti-PD-1 antibody/scFv in blocking the binding of PD-L1 to CHO cells overexpressing human PD-1

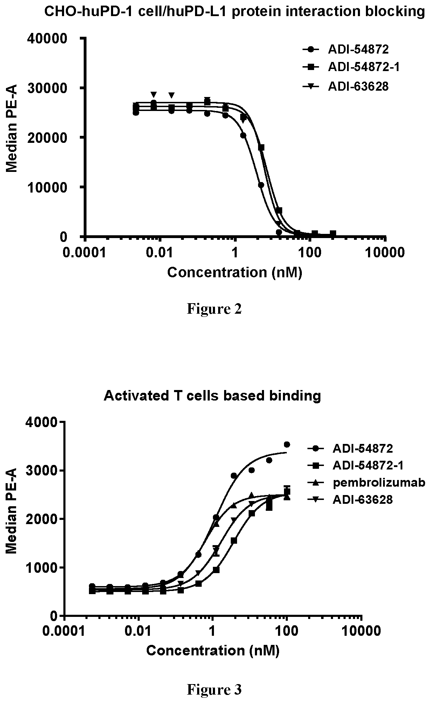

The CHO-huPD-1 cells after expansion culture was adjusted to reach a cell density of 2×10⁶/mL, added at 100 µL/well to a 96-well flow plate, and centrifuged for later use. The purified monoclonal antibody was diluted with PBS by 3-fold dilution starting from 400 nM for a total of 12 points. The diluted sample was added at 60 µL/well to the above 96-well flow plate with cells, and incubated at 4°C for 30 minutes. Then bio-human PD-L1-Fc protein was added at 60 µL/well to reach a final concentration of 0.5 µg/mL, incubated at 4°C for 30 minutes, and washing was performed twice with PBS. SA-PE diluted 100 times with PBS was added at 100 µL/well, incubated at 4°C for 30 minutes, and washing was performed twice with PBS. PBS was added at 100 µL/well to resuspend the cells, the detection was performed on a CytoFlex flow cytometer, and the corresponding MFI was calculated. The results were shown in Table 4 and Figure 2: ADI-54872, ADI-54872-1, and ADI-63628 had good blocking activity.

### Example 5: Binding of anti-PD-1 antibody/scFv to surface PD-1 of primary T cells

The binding activity of the anti-PD-1 antibody/scFv of the present invention to surface PD-1 of activated T cells was detected based on flow cytometry.

Specifically, human PBMCs were sorted according to the experimental protocol provided by STEMCELL (STEMCELL, catalog number: #17951C) to obtain human total T cells. The T cell concentration was adjusted to 1.0×10⁶/mL using X-VIVO15 culture medium (purchased from lonza, catalog number: 04-418Q), 1 µL of IL-2 stock solution (1 million IU) was added, and CD3/CD28 Dynabeads (purchased from gibco, catalog number: 11132D) were added at 1:1 (bead-to-cell), and cultured in a 37°C, 5% CO₂ incubator for 48 hours. The activated T cells were adjusted to an appropriate cell density and added to a 96-well flow plate. After centrifugation, the gradiently diluted sample to be tested was added and incubated at 4°C for 30 minutes. Washing was performed twice with PBS, the corresponding fluorescent secondary antibody diluted to an appropriate concentration was added, incubated at 4°C for 30 minutes, and washing was performed twice with PBS. The cells were resuspended in PBS, detected on a CytoFlex flow cytometer, and the corresponding MFI was calculated. The results were shown in Figure 3. The anti-PD-1 antibody/scFv ADI-54872, ADI-54872-1 and ADI-63628 of the present invention could bind to the PD-1 molecules on the surface of activated T cells, and ADI-54872 was superior to the control antibody pembrolizumab.

### Example 6: Experiment of anti-PD-1 antibody/scFv blocking PD-L1/PD-1 luciferase reporter gene

In order to further detect the blocking activity of PD-1 antibody/scFv at the cellular level, a luciferase reporter gene system was constructed in this example. In brief, cells were transfected with lentivirus to construct a CHO-K1 cell line (CHO-K1-PD-L1) overexpressing human PD-L1 and OKT-3 scFv, and a Jurkat cell line (Jurkat-PD-1-luc) overexpressing human PD-1 and NF-AT luciferase reporter gene (purchased from Promega), and the reporter gene system was subsequently used to carry out related experiments.

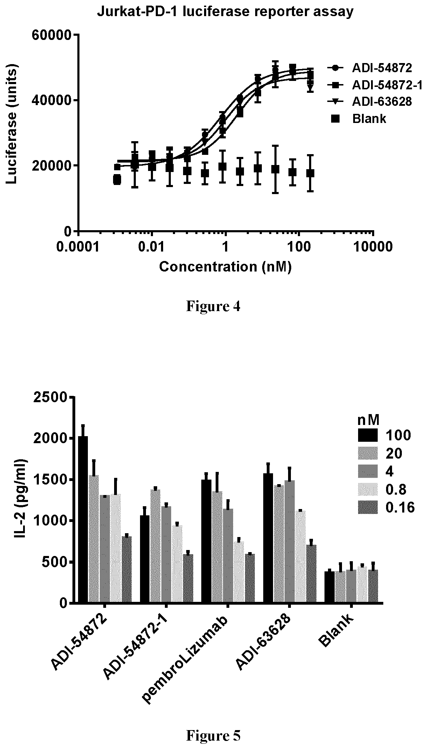

Specifically, CHO-K1-PD-L1 functional cells were obtained by digestion, adjusted to an appropriate cell density, added at 100 µL/well to a 96-well white bottom plate, and cultured overnight. On the next day, a Jurkat-PD-1-luc effector cell suspension was prepared, and the sample to be tested was gradiently diluted with a reaction medium (RPMI1640+10% FBS). The white bottom plate was taken out, the culture supernatant was removed by pipetting, the diluted sample was added at 40 µL/well to the white bottom plate, and the Jurkat-PD-1-luc effector cell suspension was added at 40 µL/well at the same time. Culture was performed in a 37°C, 5% CO₂ incubator for 6 hours, during which, Bio-Glo TM reagent (purchased from promega, catalog number: G7940) was restored to room temperature. After the culture was completed, the cells were taken out, balanced at room temperature for 5 minutes, added with the Bio-Glo TM reagent at 80 µL/well, and the fluorescence signal value was read using a multifunctional microplate reader. As shown in Figure 4, the anti-PD-1 antibody/scFv ADI-54872, ADI-54872-1, and ADI-63628 of the present invention could block the PD-L1-mediated PD-1 downstream signaling pathway and upregulate the reporter gene luciferase expression.

### Example 7: Experiment of mixed lymphocyte reaction

In this example, the activity of PD-1 antibody/scFv activating T cells was detected by the experiment of mixed lymphocyte reaction (MLR). The specific experimental method was follows.

PBMC cells (purchased from SAILY BIO, SLB-HPB) were resuscitated and centrifuged. PBMC were resuspended in 10 ml of X-VIVO-15 medium (purchased from LONZA), cultured in a cell culture incubator at 37°C for 2 h, and non-adherent cells were removed by pipetting. 10 ml of DC culture medium was added: X-VIVO-15 culture medium was added with 10 ng/ml GM-CSF (purchased from R&D), 20 ng/ml IL-4, cultured for 3 days, added with 5 ml of DC culture medium, and continuously cultured until the 6th day; DC maturation culture medium was added: X-VIVO-15 culture medium was added with 1000 U/ml TNF-α (purchased from R&D), 10 ng/ml IL-6 (purchased from R&D), 5 ng/ml IL-1β (purchased from R&D), 1 µM PGE2 (purchased from Tocris), and cultured for 2 days, and the mature DC cells were collected, and adjusted with X-VIVO-15 culture medium to reach a cell density of 2×10⁵/ml.

The PBMC cells from another donor (purchased from SAILY BIO, SLB-HPB) were Resuscitated, centrifuged. The PBMC were resuspended with 10 ml of X-VIVO-15 culture medium. The total T cells were enriched with a total T cell sorting kit (purchased from Stemcell), and the total T cells were resuspended with X-VIVO-15 to adjust the cell density to 2×10⁶/ml. The T cells were mixed with the above-collected mature DC cells at a ratio of 1:1, and added at 100 µl/well to a 96-well U-bottom plate.

PD-1 antibody/scFv was diluted with X-VIVO-15 medium by 5-fold dilution starting from 200 nM for a total of 5 points, added at 100 µl/well to the above-mentioned mixed cell wells, cultured for 3 days, and the supernatant was collected. ELISA (purchased from eBioscience) method was used to detect the expression level of IL2.

The results were shown in Figure 5. The anti-PD-1 antibody/scFv ADI-54872, ADI-54872-1, and ADI-63628 of the present invention could activate T cells to secrete IL-2 in the MLR experiment, and the activation activity of ADI-54872 was better than that of the control antibody nembrolizumab

### Example 8: In vivo pharmacodynamic study of anti-PD-1 antibody in human PD-1 transgenic mice

In this experiment, human PD-L1 KI MC38 tumor cells (MC38-huPD-L1 cells) were subcutaneously inoculated in human PD-1 transgenic C57 mice (huPD-1 KI mice) to determine the anti-tumor effect of the PD-1 antibody of the present invention.

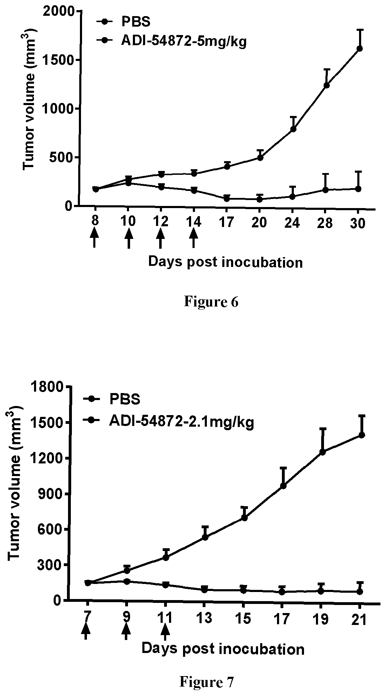

Specifically, the MC38-huPD-L1 cell tumor-bearing mouse model was first established by subcutaneous inoculation. When the average tumor volume grew to about 173 mm³, the mice were grouped and intraperitoneally injected with PBS, 5 mg/kg ADI-54872, and 5 mg/kg Pembrolizumab for treatment. The tumor volume and body weight changes of each group of mice were monitored. The monitoring frequency was once per 2 to 4 days, and the monitoring was carried out for consecutive 3 weeks. The dosage and method of administration were shown in Table 5. The results were shown in Figure 6. The anti-PD-1 antibody ADI-54872 of the present invention had significant anti-tumor activity.

**Table 5: Dosage regimen of anti-PD-1 antibody**

| Group | Dosage | Administration frequency/number of times |
|---|---|---|
| Negative control (PBS) | N/A | Q2d×4 |
| ADI-54872 | 5 mg/kg | Q2d×4 |

### Example 9: In vivo pharmacodynamic study of anti-PD-1 antibody in B-NDG mice inoculated with A375 tumor cells and human PBMC

In this experiment, the anti-tumor activity of the anti-PD-1 antibody of the present invention in a tumor model in which B-NDG mice were inoculated with A375 tumor cells and human PBMC

Specifically, A375 tumor-bearing mouse model was first established by subcutaneous mixed inoculation of A375 tumor cells + human PBMC. When the average tumor volume grew to about 147 mm³, the mice were grouped, and treated with PBS, 2.1 mg/kg ADI-54872, and 2.1 mg/kg Pembrolizumab by intraperitoneal injection. The changes in tumor volume and body weight of mice in each group were monitored. The monitoring frequency was once per 2 days, and the monitoring was continued for 2 weeks. The dosage and method of administration were shown in Table 6. As shown in Figure 7, the anti-PD-1 antibody ADI-54872 of the present invention had significant anti-tumor activity.

**Table 6: Dosage regimen of anti-PD-1 antibody**

| Group | Dosage | Administration frequency/number of times |
|---|---|---|
| Negative control (PBS) | N/A | Q2d×3 |
| ADI-54872 | 2.1 mg/kg | Q2d×3 |

### Example 10: Cloning and expression of anti-PD-1/TGF-βtrap fusion protein

In this example, TGF-βR2 extracellular domain (SEQ ID NO: 19) was used as the immunomodulatory molecule part of fusion protein, and the PD-1 antibody was used as the targeting part of fusion protein to form a PD-1 antibody/TGF-βRII extracellular region fusion protein, named 54872-TGF-βRII.

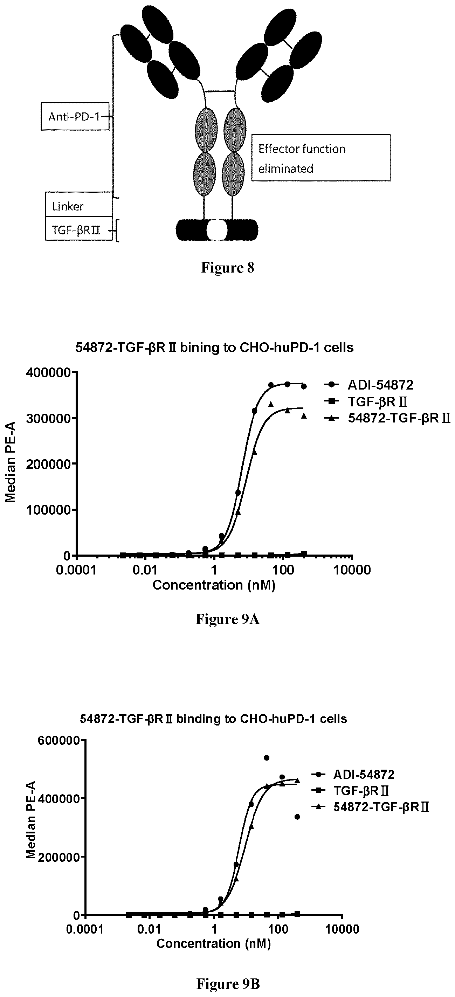

The heavy chain C-terminal amino acid of ADI-54872 was connected to the TGF-βRII extracellular region through (G₄A)₄G by molecular cloning technology, and conventional expression was performed by the Expi-CHO expression system. The expression and purification method was the same as in Example 1, and the fusion protein 54872-TGF-βRII with the structure shown in Figure 8 was obtained. The full-length heavy chain sequence and light chain sequence were set forth in SEQ ID NO: 17 and SEQ ID NO: 18, respectively.

### Example 11: Binding activity of 54872-TGF-βRII molecule to CHO cells overexpressing human/cynomolgus monkey PD-1

The method for detecting the binding activity of the purified PD-1 antibody ADI-54872, 54872-TGF-βRII molecules, TGF-βR2-Fc fusion protein to cell surface PD-1 was the same as in Example 3. In the detection experiment of the above method, the experimental results were shown in Figures 9A to 9B, and the 54872-TGF-βRII molecule of the present invention had binding activity to CHO cells overexpressing human PD-1 and cynomolgus monkey PD-1.

### Example 12: Activity of 54872-TGF-βRII molecule blocking the binding of PD-L1 to CHO cells overexpressing human PD-1

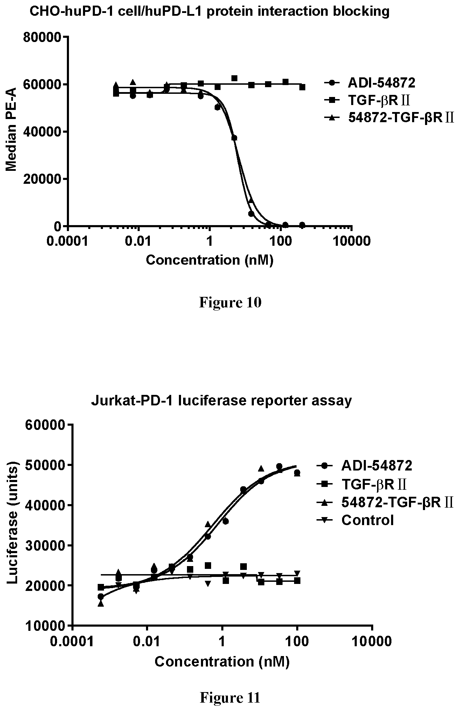

The method for detecting the activity of the purified PD-1 antibody ADI-54872, 54872-TGF-βRII molecule, and TGF-βR2-Fc fusion protein in blocking the binding of PD-L1 to CHO cells overexpressing human PD-1 was the same as that in Example 4. In the detection experiment of the above method, the experimental results were shown in Figure 10, and the 54872-TGF-βRII molecule of the present invention could block the binding of PD-L1 to CHO cells overexpressing human PD-1.

### Example 13: Experiment of 54872-TGF-βRII molecule blocking PD-L1/PD-1 luciferase reporter gene

The method for detecting the purified PD-1 antibody ADI-54872, 54872-TGF-βRII molecule, TGF-βR2-Fc fusion protein and negative control to block the PD-1 downstream signaling pathway mediated by PD-L1 at the cellular level was the same as that in Example 6. In the above detection experiment, the experimental results were shown in Figure 11. The 54872-TGF-βRII molecule of the present invention could block the PD-1 downstream signaling pathway mediated by PD-L1 and upregulate the reporter gene luciferase expression.

### Example 14: Experiment of 54872-TGF-βRII molecule binding to human TGF-β family protein at ELISA level

Human TGF-β1 (Acrobiosystems, TG1-H421), TGF-β2 (PeproTech, 100-35B), and TGF-β3 (PeproTech, 100-36E) proteins were diluted with ELISA coating solution and added to an ELISA plate, and coated overnight at 4°C. The coating solution was discarded, the plated was washed 3 times by adding PBST at 250 µL/well, and blocked with 5% BSA at room temperature for 1 hour for later use. The purified PD-1 antibody ADI-54872, 54872-TGF-βRII molecule, TGF-βR2-Fc fusion protein, and negative control were gradiently diluted with 1% BSA and added to the blocked ELISA plate, and incubated at room temperature for 2 hours. The plate was washed 3 times by adding PBST, added to the wells with mouse anti-human Fc-HRP (SouthernBiotech, 9040-05), incubated at room temperature for 1 hour, washed 3 times by adding PBST, then added with an ELISA color development solution, placed at room temperature for 3 min, and added with an ELISA stop solution, and the absorbance value at 450 nm was read.

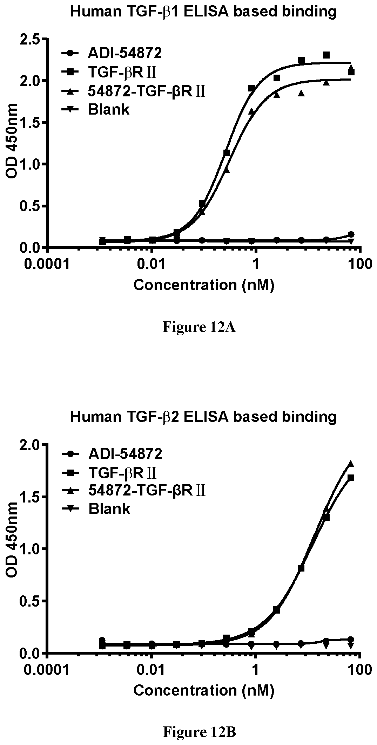

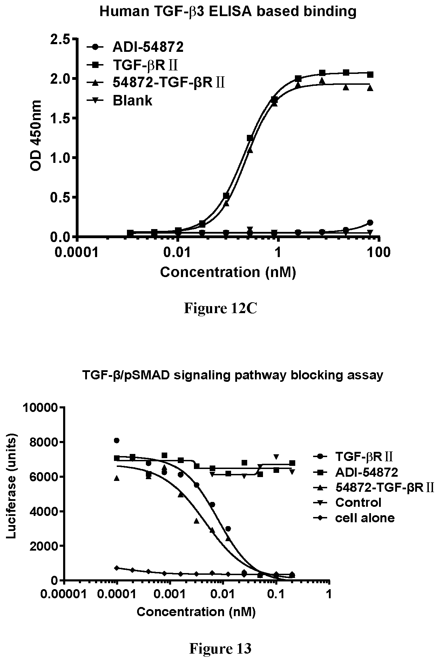

In the detection experiment of the above method, the experimental results were shown in Figures 12A to 12C. The 54872-TGF-βRII molecule of the present invention had good binding to human TGF-β1 and TGF-β3 proteins at the ELISA level, and weak binding activity to human TGF-β2 protein.

### Example 15: Experiment of 54872-TGF-βRII molecule blocking TGF-β/SMAD signaling pathway

An appropriate amount of 293-TGF-β/SMAD effector cells were taken and inoculated in a 96-well cell culture white bottom plate, and cultured it in a 37°C, 5% CO₂ incubator overnight. The purified PD-1 antibody ADI-54872, 54872-TGF-βRII molecule, TGF-βR2-Fc fusion protein, and negative control were gradiently diluted and mixed with TGF-β1 (Acrobiosystems, TG1-H421), and incubated at room temperature for 30 min. The above mixture was added to the white bottom plate with cells and continuously cultured overnight. Bio-Glo TM reagent (Promega) was added to each well, and the fluorescence signal value was read using a multifunctional microplate reader.

In the detection experiment of the above method, the experimental results were shown in Figure 13. The 54872-TGF-βRII molecule of the present invention could block the TGF-β/SMAD signaling pathway in vitro.

### Example 16: Experiment of 54872-TGF-βRII molecule ELISA co-binding to human TGF-β1/PD-1 protein at ELISA level

Human TGF-β1 (Acrobiosystems, TG1-H421) or human PD-1 protein was diluted with ELISA coating solution, then added to an ELISA plate, and coated at 4°C overnight. The coating solution was discarded, the plate was washed 3 times by adding PBST at 250 µL/well, and blocked with 5% BSA at room temperature for 1 hour for later use. The purified PD-1 antibody ADI-54872, 54872-TGF-βRII molecule, TGF-βR2-Fc fusion protein, and negative control were gradiently diluted with 1% BSA, then added to the blocked ELISA plate, and incubated at room temperature for 2 hours. Washing was performed 3 times with PBST, Biotin-PD-1 or Biotin-Anti-TGFβR2 was added to the wells, and incubated at room temperature for 1 hour. Washing was performed 3 times with PBST, Streptavidin-HRP (abcam, ab7403) was added to the wells, incubated at room temperature for half an hour, and washing was performed 3 times with PBST. An ELISA color development solution was added, allowed to stand at room temperature for 3 min, then an ELISA stop solution was added, and the absorbance value at 450 nm was read.

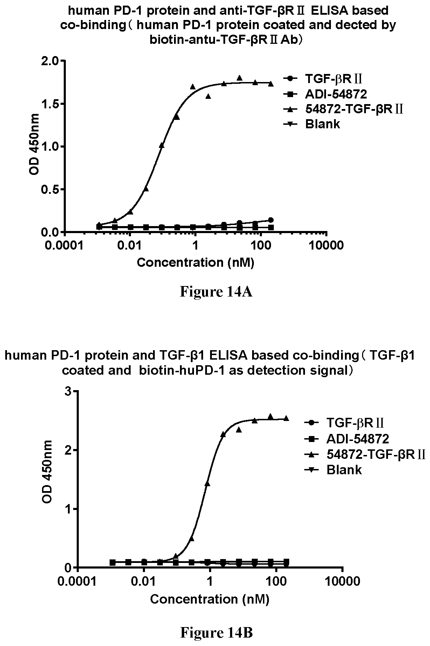

In the detection experiment of the above method, the experimental results were shown in Figures 14A to 14B. The 54872-TGF-βRII molecule of the present invention could co-bind to human TGF-β1 and PD-1 proteins at the ELISA level.

### Example 17: Binding activity of 54872-TGF-βRII molecule to CHO cells overexpressing human PD-1 in the presence of human TGF-β1 protein

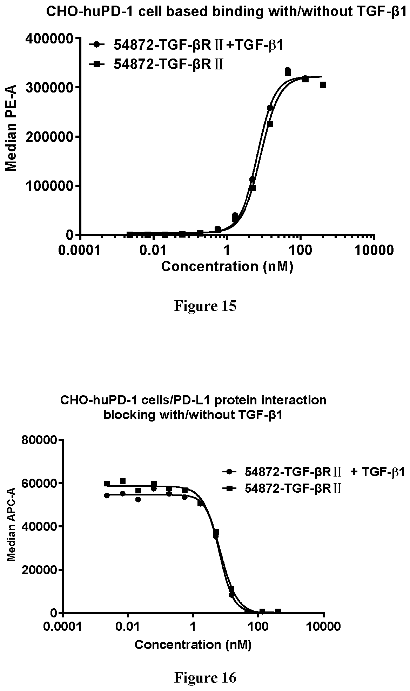

After gradient dilution of 54872-TGF-βRII molecule, an equal volume of human TGF-β1 protein was added and incubated at room temperature for 30 minutes. CHO cells overexpressing human PD-1 after expansion culture were adjusted to an appropriate cell density and added to a 96-well flow plate. After centrifugation, the above-incubated sample was added, and incubated at 4°C for 30 minutes. The subsequent detection method was the same as that in Example 3. In the determination experiment of the above method, the experimental results were shown in Figure 15. The presence of human TGF-β1 protein did not affect the binding activity of the 54872-TGF-βRII molecule of the present invention to CHO cells overexpressing human PD-1.

### Example 18: Activity of 54872-TGF-βRII molecule blocking the binding of PD-L1 to CHO cells overexpressing human PD-1 in the presence of human TGF-β1 protein

After gradient dilution of 54872-TGF-βRII molecule, an equal volume of human TGF-β1 protein was added, and incubated at room temperature for 30 minutes. CHO cells overexpressing human PD-1 after expansion culture were adjusted to an appropriate cell density and added to a 96-well flow plate. After centrifugation, the above-incubated sample was added and incubated at 4°C for 30 minutes. The subsequent detection method was the same as that in Example 4. In the determination experiment of the above method, the experimental results were shown in Figure 16. The presence of human TGF-β1 protein did not affect the activity of the 54872-TGF-βRII molecule of the present invention in blocking the binding of PD-L1 to CHO cells overexpressing human PD-1.

### Example 19: Experiment of mixed lymphocyte reaction

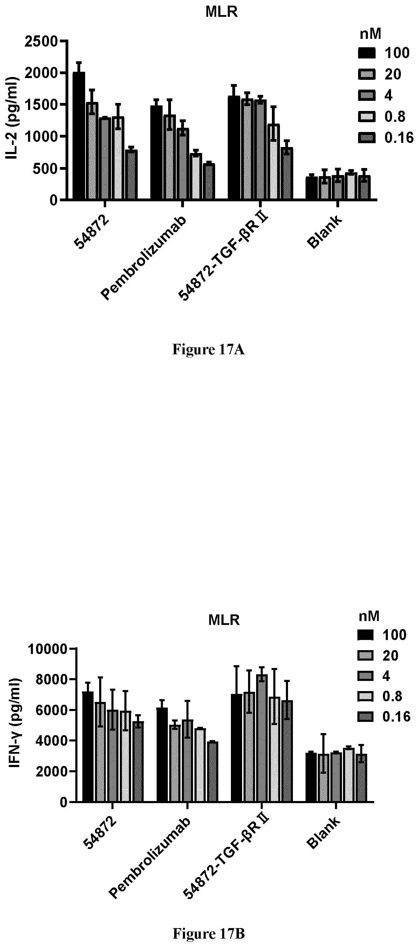

The method for mixed lymphocyte detection of the purified PD-1 antibody ADI-54872, 54872-TGF-βRII molecule, TGF-βR2-Fc fusion protein, and negative control to activate human T lymphocytes was the same as that in Example 7. The results were shown in Figures 17A to 17B. The 54872-TGF-βRII molecule of the present invention could activate T cells to secrete IL-2 and IFN-γ in the MLR experiment.

### Example 20: In vivo pharmacodynamic study of 54872-TGF-βRII molecule in B-NDG mice inoculated with A375 tumor cells and human PBMC

In this experiment, the anti-tumor activity of the anti-PD-1 antibody of the present invention in a tumor model in which B-NDG mice were inoculated with A375 tumor cells and human PBMC.

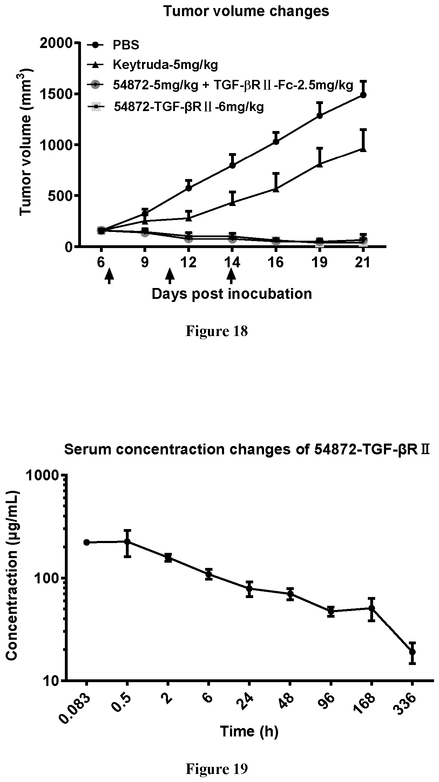

Specifically, the A375 tumor-bearing mouse model was first established by subcutaneous mixed inoculation of A375 tumor cells + human PBMC. When the average tumor volume grew to about 150 mm³, the mice were divided into groups, and treated by intraperitoneal injection of PBS, 5 mg/kg Keytruda (Pembrolizumab), 5 mg/kg ADI-54872 combined with 2.5 mg/kg TGF-βRII-Fc, and 6 mg/kg 54872-TGF-βRII (equivalent molar dose to 5 mg/kg Keytruda). The tumor volume and body weight changes of each group of mice were monitored. The monitoring frequency was once per 2 to 3 days, and the monitoring was performed continuously for 2 to 3 weeks. The dosage and method of administration were shown in Table 7. The results were shown in Figure 18. The anti-tumor activity of the 54872-TGF-βRII molecule of the present invention was better than that of the Keytruda with an equal molar dose.

**Table 7: Dosage regimen of 54872-TGF-βRII molecule**

| Group | Dosage | Administration frequency/number of times |
|---|---|---|
| Negative control (PBS) | N/A | Q3d×3 |
| Keytruda | 5 mg/kg | Q3d×3 |
| ADI-54872+TGF-βRII-Fc- | 5 + 2.5mg/kg | Q3d×3 |
| 54872-TGF-βRII | 6 mg/kg | Q3d×3 |

### Example 21: Pharmacokinetic evaluation of 54872-TGF-βRII molecule in mice

18 BALB/C mice were used in the experiment, half male and half female, underwent 12/12 hours light/dark adjustment, at temperature 24±2°C, and humidity 40-70%, with free access to water and diet. The mice were purchased from Zhejiang Weitong Lihua Experimental Technology Co., Ltd. On the day of the experiment, the mice were injected with 54872-TGF-βRII molecule once through the tail vein, with an injection dose of 10 mg/kg.

Serum collection time points: 5 minutes, 0.5 hours, 2 hours, 6 hours, 24 hours, 48 hours, 96 hours, 168 hours, and 336 hours after the administration, blood was collected from the mouse orbital vein. The whole blood sample was placed at 2-8°C for 30 minutes, centrifuged at 12000 rpm for 5 minutes to collect serum, and the obtained serum was centrifuged at 2-8°C and 12000 rpm for 5 minutes, stored at -80°C, and ELISA was used to detect the molecular weight of free 54872-TGF-βRII in the serum. The results were shown in Figure 19 and Table 8. The half-life of the free state molecule of 54872-TGF-βRII in BALB/C mice was about 160 hours.

**Table 8: T1/2 of 54872-TGF-βRII in mice**

| Drug to be tested | Administration method | T1/2 |
|---|---|---|
| 54872-TGF-βRII | IV | 160 hours |

Although the specific embodiments of the present invention have been described in detail, those skilled in the art will understand that various modifications and changes can be made to the details based on all the teachings disclosed, and these changes are within the scope of protection of the present invention. The entirety of the present invention is given by the appended claims and any equivalents thereof.

## Claims

1. An antibody or an antigen-binding fragment thereof capable of specifically binding to PD-1, the antibody or antigen-binding fragment thereof comprising:
(i) a heavy chain variable region (VH) comprising the following 3 complementary determining regions (CDRs): VH CDR1 having a sequence as set forth in SEQ ID NO: 7 or 13, VH CDR2 having a sequence as set forth in SEQ ID NO: 8, and VH CDR3 having a sequence as set forth in SEQ ID NO: 9; and/or,
a light chain variable region (VL) comprising the following 3 complementary determining regions (CDRs): VL CDR1 having a sequence as set forth in SEQ ID NO: 10, VL CDR2 having a sequence as set forth in SEQ ID NO: 11 or 14, and VL CDR3 having a sequence as set forth in SEQ ID NO: 12;
or
(ii) 3 CDRs as contained in the VH as set forth in SEQ ID NO: 1, 3 or 5, and/or, 3 CDRs as contained in the VL as set forth in SEQ ID NO: 2, 4 or 6; preferably, the CDRs are determined by the Kabat numbering system, the Chothia numbering system, or the IMGT numbering system.

2. The antibody or antigen-binding fragment thereof according to claim 1, wherein the antibody or antigen-binding fragment thereof comprises:
(a) a heavy chain variable region (VH) comprising the following 3 complementary determining regions (CDRs): VH CDR1 having a sequence as set forth in SEQ ID NO: 7, VH CDR2 having a sequence as set forth in SEQ ID NO: 8, and VH CDR3 having a sequence as set forth in SEQ ID NO: 9; and/or, a light chain variable region (VL) comprising the following 3 complementary determining regions (CDRs): VL CDR1 having a sequence as set forth in SEQ ID NO: 10, VL CDR2 having a sequence as set forth in SEQ ID NO: 11, and VL CDR3 having a sequence as set forth in SEQ ID NO: 12;
or
(b) a heavy chain variable region (VH) comprising the following 3 complementary determining regions (CDRs): VH CDR1 having a sequence as set forth in SEQ ID NO: 13, VH CDR2 having a sequence as set forth in SEQ ID NO: 8, and VH CDR3 having a sequence as set forth in SEQ ID NO: 9; and/or, a light chain variable region (VL) comprising the following 3 complementary determining regions (CDRs): VL CDR1 having a sequence as set forth in SEQ ID NO: 10, VL CDR2 having a sequence as set forth in SEQ ID NO: 14, and VL CDR3 having a sequence as set forth in SEQ ID NO: 12.

3. The antibody or antigen-binding fragment thereof according to claim 1 or 2, wherein the antibody or antigen-binding fragment thereof comprises: a heavy chain variable region (VH) comprising a sequence as set forth in SEQ ID NO: 1, 3 or 5 or variant thereof; and/or, a light chain variable region (VL) comprising a sequence as set forth in SEQ ID NO: 2, 4 or 6 or variant thereof;
wherein, the variant has a substitution, deletion or addition of one or more amino acids (e.g., a substitution, deletion or addition of 1, 2, 3, 4 or 5 amino acids), or a sequence having a sequence identity of at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived; preferably, the substitution is a conservative substitution.

4. The antibody or antigen-binding fragment thereof according to any one of claims 1 to 3, comprising:
(a) a heavy chain variable region (VH) comprising a sequence as set forth in SEQ ID NO: 1 or 3 or variant thereof; and/or, a light chain variable region (VL) comprising a sequence as set forth in SEQ ID NO: 2 or 4 or variant thereof; or,
(b) a heavy chain variable region (VH) comprising a sequence as set forth in SEQ ID NO: 5 or variant thereof; and/or, a light chain variable region (VL) comprising a sequence as set forth in SEQ ID NO: 6 or variant thereof;
wherein, the variant has a substitution, deletion or addition of one or more amino acids (e.g., a substitution, deletion or addition of 1, 2, 3, 4 or 5 amino acids), or a sequence having a sequence identity of at least 80%, at least 85%, at least 90%, at least 91%, at least 92%, at least 93%, at least 94%, at least 95%, at least 96%, at least 97%, at least 98%, at least 99%, or 100%, as compared to the sequence from which it is derived; preferably, the substitution is a conservative substitution.

5. The antibody or antigen-binding fragment thereof according to any one of claims 1 to 4, comprising:
(1) a VH comprising the sequence as set forth in SEQ ID NO: 1 and a VL comprising the sequence as set forth in SEQ ID NO: 2;
(2) a VH comprising the sequence as set forth in SEQ ID NO: 3 and a VL comprising the sequence as set forth in SEQ ID NO: 4; or,
(3) a VH comprising the sequence as set forth in SEQ ID NO: 5 and a VL comprising the sequence as set forth in SEQ ID NO: 6.

6. The antibody or antigen-binding fragment thereof according to any one of claims 1 to 5, further comprising a constant region derived from a human immunoglobulin;
preferably, the heavy chain of the antibody or antigen-binding fragment thereof comprises a heavy chain constant region derived from a human immunoglobulin (e.g., IgG1, IgG2, IgG3, or IgG4), and the light chain of the antibody or antigen-binding fragment thereof comprises a light chain constant region derived from a human immunoglobulin (e.g., κ or λ);
preferably, the antibody or antigen-binding fragment thereof comprises a heavy chain constant region (CH) as set forth in SEQ ID NO: 15 and/or a light chain constant region (CL) as set forth in SEQ ID NO: 16.

7. The antibody or antigen-binding fragment thereof according to any one of claims 1 to 6, wherein the antigen-binding fragment is selected from the group consisting of Fab, Fab', (Fab')₂, Fv, disulfide-bonded Fv, scFv, diabody, chimeric antibody, humanized antibody, bispecific antibody, or multispecific antibody.

8. The antibody or antigen-binding fragment thereof according to any one of claims 1 to 7, wherein the antibody is an IgG antibody (e.g., an IgG1, IgG2, IgG3 or IgG4 antibody).

9. The antibody or antigen-binding fragment thereof according to any one of claims 1 to 8, wherein the antibody or antigen-binding fragment thereof is labeled; preferably, the antibody or antigen-binding fragment thereof bears a detectable label, such as an enzyme (e.g., horseradish peroxidase or alkaline phosphatase), a chemiluminescent agent (e.g., acridinium ester compound, luminol and derivative thereof, or ruthenium derivative), a fluorescent dye (e.g., fluorescein or fluorescent protein), a radionuclide or a biotin.

10. A fusion protein, which comprises the antibody or antigen-binding fragment thereof according to any one of claims 1 to 9 and an additional biologically active polypeptide.

11. The fusion protein according to claim 10, wherein the additional biologically active polypeptide is an immunomodulator.

12. The fusion protein according to claim 11, wherein the additional biologically active polypeptide is a TGF-β/TGF-βR pathway inhibitor, such as a TGF-βRII extracellular domain.

13. The fusion protein according to any one of claims 10 to 12, wherein the antibody or antigen-binding fragment thereof is optionally linked to the additional biologically active polypeptide via a peptide linker.

14. The fusion protein according to any one of claims 10-13, comprising:
a first peptide chain comprising a light chain of the antibody or antigen-binding fragment thereof: and
a second peptide chain comprising a heavy chain of the antibody or antigen-binding fragment thereof and the additional biologically active polypeptide.

15. The fusion protein according to claim 14, wherein the first peptide chain comprises a sequence as set forth in SEQ ID NO: 17, and/or, the second peptide chain comprises a sequence as set forth in SEQ ID NO: 18.

16. A polypeptide construct, which comprises the antibody or antigen-binding fragment thereof according to any one of claims 1 to 9, and an immunoglobulin Fc domain;
preferably, the antibody or antigen-binding fragment thereof is an antigen-binding fragment, such as Fab, Fab', (Fab')₂, Fv, disulfide-linked Fv, scFv or diabody.

17. The polypeptide construct according to claim 16, wherein the antibody or antigen-binding fragment thereof is a scFv;
preferably, the scFv comprises a disulfide bond;
preferably, the additional biologically active polypeptide is optionally linked to the N-terminal of the scFv via a peptide linker.

18. The polypeptide construct according to claim 17, comprising a sequence as shown in any one of SEQ ID NO: 23 or 24.

19. An isolated nucleic acid molecule, which encodes the antibody or antigen-binding fragment thereof according to any one of claims 1 to 9, or a heavy chain variable region and/or light chain variable region thereof, or encodes the fusion protein according to any one of claims 10 to 15, or encodes the polypeptide construct according to any one of claims 16 to 18.

20. A vector, which comprises the nucleic acid molecule according to claim 19; preferably, the vector is a cloning vector or an expression vector.

21. A host cell, which comprises the nucleic acid molecule according to claim 19 or the vector according to claim 20.

22. A method for preparing the antibody or antigen-binding fragment thereof according to any one of claims 1 to 9 or the fusion protein according to any one of claims 10 to 15 or the polypeptide construct according to any one of claims 16 to 18, comprising culturing the host cell according to claim 21 under conditions that allow protein expression, and recovering the antibody or antigen-binding fragment thereof or the fusion protein or the polypeptide construct from a culture of the cultured host cell.

23. A conjugate, which comprises the antibody or antigen-binding fragment thereof according to any one of claims 1 to 9 and a therapeutic agent linked to the antibody or antigen-binding fragment thereof;
preferably, the therapeutic agent is selected from the group consisting of cytotoxin or radioactive isotope.

24. A composition, which comprises: (1) the antibody or antigen-binding fragment thereof according to any one of claims 1 to 9 or the polypeptide construct according to any one of claims 16 to 18; and (2) an immunomodulator.

25. The composition according to claim 24, wherein the immunomodulator is a TGF-β/TGF-βR pathway inhibitor, such as a TGF-βRII extracellular domain.

26. A pharmaceutical composition, which comprises the antibody or antigen-binding fragment thereof according to any one of claims 1 to 9, the fusion protein according to any one of claims 10 to 15, the polypeptide construct according to any one of claims 16 to 18, the conjugate according to claim 23, or the composition according to claim 24 or 25, and a pharmaceutically acceptable carrier and/or excipient;
preferably, the pharmaceutical composition further comprises an additional pharmaceutically active agent;
preferably, the additional pharmaceutically active agent is a drug with anti-tumor activity, such as an additional immune checkpoint inhibitor, an oncolytic virus, a chemotherapeutic agent, an anti-angiogenic drug, an antimetabolite drug, a tumor-targeting drug or an immunostimulant;
preferably, the additional pharmaceutically active agent is a drug for treating an infection, such as an antiviral agent, an antifungal agent, an antibacterial agent or an immune stimulant;
preferably, the antibody or antigen-binding fragment thereof, the fusion protein, the conjugate or the composition and the additional pharmaceutically active agent are provided as separate components or as components of the same composition.

27. Use of the antibody or antigen-binding fragment thereof according to any one of claims 1 to 9, the fusion protein according to any one of claims 10 to 15, the polypeptide construct according to any one of claims 16 to 18, the conjugate according to claim 23, the composition according to claim 24 or 25, or the pharmaceutical composition according to claim 26, in the manufacture of a medicament for:
(1) increasing an immune cell activity in vitro or in vivo in a subject (e.g., a human);
(2) enhancing an immune response in a subject (e.g., a human);
(3) preventing and/or treating a tumor in a subject (e.g., a human);
(4) preventing and/or treating an infection in a subject (e.g., a human);
preferably, the tumor is a tumor with microsatellite high instability (MSI-H) and/or mismatch repair deficiency (dMMR);
preferably, the tumor is a solid tumor, such as melanoma (e.g., metastatic malignant melanoma), breast cancer, renal cancer (e.g., clear cell carcinoma), prostate cancer, bladder cancer, pancreatic cancer, lung cancer (e.g., non-small cell lung cancer), colon cancer, esophageal cancer, head and neck squamous cell carcinoma, liver cancer, ovarian cancer, cervical cancer, thyroid cancer, glioblastoma, glioma;
preferably, the tumor is a blood tumor, such as lymphoma, leukemia; preferably, the lymphoma is Hodgkin's lymphoma or non-Hodgkin's lymphoma; preferably, the non-Hodgkin's lymphoma is one or more of peripheral T-cell lymphoma, angioimmunoblastic T-cell lymphoma, NK/T-cell lymphoma (nasal type) with Epstein-Barr virus positivity, and B-cell non-Hodgkin's lymphoma;
preferably, the infection is selected from the group consisting of viral infection, bacterial infection, fungal infection and parasitic infection;
preferably, the subject is a mammal, such as a human.

28. A method for enhancing an immune response, and/or preventing and/or treating a tumor or infection, in a subject; the method comprising: administering to the subject in need thereof an effective amount of the antibody or antigen-binding fragment thereof according to any one of claims 1 to 9, the fusion protein according to any one of claims 10 to 15, the polypeptide construct according to any one of claims 16 to 18, the conjugate according to claim 23, or the composition according to claim 24 or 25, or the pharmaceutical composition according to claim 26;
preferably, the tumor is a tumor with microsatellite high instability (MSI-H) and/or mismatch repair deficiency (dMMR);
preferably, the tumor is a solid tumor, such as a melanoma (e.g., metastatic malignant melanoma), breast cancer, kidney cancer (e.g., clear cell carcinoma), prostate cancer, bladder cancer, pancreatic cancer, lung cancer (e.g., non-small cell lung cancer), colon cancer, esophageal cancer, head and neck squamous cell carcinoma, liver cancer, ovarian cancer, cervical cancer, thyroid cancer, glioblastoma, glioma;
preferably, the tumor is a blood tumor, such as lymphoma, leukemia; preferably, the lymphoma is Hodgkin's lymphoma or non-Hodgkin's lymphoma; preferably, the non-Hodgkin's lymphoma is one or more of peripheral T-cell lymphoma, angioimmunoblastic T-cell lymphoma, NK/T-cell lymphoma (nasal type) with Epstein-Barr virus positivity, and B-cell non-Hodgkin's lymphoma;
preferably, the infection is selected from the group consisting of viral infection, bacterial infection, fungal infection and parasitic infection;
preferably, the subject is a mammal, such as a human.

29. A kit, which comprises the antibody or antigen-binding fragment thereof according to any one of claims 1 to 9;
preferably, the kit comprises the antibody or antigen-binding fragment thereof according to claim 9;
preferably, the kit comprises the antibody or antigen-binding fragment thereof according to any one of claims 1 to 8, and a second antibody capable of specifically recognizing the antibody or antigen-binding fragment thereof; optionally, the second antibody further comprises a detectable label, such as an enzyme (e.g., horseradish peroxidase or alkaline phosphatase), a chemiluminescent agent (e.g., acridinium ester compound, luminol and derivative thereof, or ruthenium derivative), a fluorescent dye (e.g., fluorescein or fluorescent protein), a radionuclide or a biotin.

30. A method for detecting the presence or level of PD-1 in a sample, comprising using the antibody or antigen-binding fragment thereof according to any one of claims 1 to 9;
preferably, the method is an immunological assay, such as immunoblotting, enzyme immunoassay (e.g., ELISA), chemiluminescent immunoassay, fluorescent immunoassay or radioimmunoassay;
preferably, the method comprises using the antibody or antigen-binding fragment thereof according to claim 9;
preferably, the method comprises using the antibody or antigen-binding fragment thereof according to any one of claims 1 to 8, and the method further comprises using a second antibody bearing a detectable label (e.g., an enzyme (e.g., horseradish peroxidase or alkaline phosphatase), a chemiluminescent agent (e.g., acridinium ester compound, luminol and derivative thereof, or ruthenium derivative), a fluorescent dye (e.g., fluorescein or fluorescent protein), a radionuclide or a biotin) to detect the antibody or antigen-binding fragment thereof;
preferably, the sample is a cell sample (e.g., a tumor cell) or a tissue sample (e.g., a tumor tissue) from a subject (e.g., a mammal, such as a human).

31. Use of the antibody or antigen-binding fragment thereof according to any one of claims 1 to 9 in the manufacture of a detection reagent for detecting the presence or level of PD-1 in a sample;
preferably, the detection reagent detects the presence or level of PD-1 in the sample by the method according to claim 30;
preferably, the sample is a cell sample (e.g., a tumor cell) from a subject (e.g., a mammal, e.g., a human).
